# EUROPEAN PATENT APPLICATION

(11) **EP 4 759 816 A1**
(43) Date of publication of application: **17.06.2026**
(21) Application number: 24850862.4
(22) Date of filing: 30.07.2024
(51) Int. Cl.: C07D 487/14, A61K 31/437, A61P 31/04

(54) **UREA-CONTAINING TRICYCLIC ß-LACTAMASE INHIBITOR, PREPARATION METHOD THEREFOR AND USE THEREOF**

(30) Priority: 08.08.2023 CN 202310992677
(71) Applicant: CHENGDU TETRAHEDRAL DRUG RESEARCH CO., LTD, Sichuan 610096 (CN)
(72) Inventor: YU, Luoting, Chengdu, Sichuan 610096 (CN); ZHANG, Xueqing, Chengdu, Sichuan 610096 (CN); WANG, Ningyu, Chengdu, Sichuan 610096 (CN); GAO, Chao, Chengdu, Sichuan 610096 (CN); ZHU, Jin, Chengdu, Sichuan 610096 (CN); LIU, Yang, Chengdu, Sichuan 610096 (CN); SHI, Jixiang, Chengdu, Sichuan 610096 (CN); REN, Hui, Chengdu, Sichuan 610096 (CN); YING, Xianli, Chengdu, Sichuan 610096 (CN)
(74) Representative: Crow, Martin
(86) International application number: PCT/CN2024/108495
(87) International publication number: WO 2025/031214

(57) **Abstract**

The present invention provides a tricyclic urea-containing β-lactamase inhibitor, a preparation method therefor, and a use thereof, belonging to the field of medicinal chemistry. The tricyclic urea-containing β-lactamase inhibitor of the present invention is a compound represented by Formula I, or a pharmaceutically acceptable salt, a stereoisomer, or a solvate thereof. The present invention provides a class of β-lactamase inhibitors with entirely novel structures. When these molecules are used in combination with traditional β-lactam antibiotics, they can reverse the resistance of most bacteria to β-lactam antibiotics caused by the expression of β-lactamases, showing promising application prospects in the clinical treatment of bacterial infections.

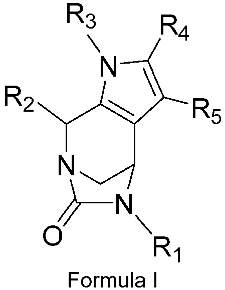

## Description

### TECHNICAL FIELD

The present disclosure relates generally to the field of medicinal chemistry, and specifically relates to urea-containing tricyclic β-lactamase inhibitor, preparation method therefor and use thereof.

### BACKGROUND

Since Fleming discovered penicillin in the 1920s, the invention and clinical application of antibiotics have profoundly changed human healthcare and quality of life, greatly increasing the average life expectancy of populations worldwide. However, the resistance of pathogenic bacteria to antibiotics in use was observed as a scientific fact very early on. Reports of penicillin-resistant Streptococcus pneumoniae isolates emerged in 1967; subsequently, penicillinase-producing Neisseria gonorrhoeae isolates were found in 1976; extended-spectrum cephalosporins such as cefotaxime were introduced in 1980, and within three years, extended-spectrum beta-lactamase-producing Escherichia coli isolates were identified. Over time and with the continuous introduction of new varieties into the clinic, especially with the expanded use of antibiotics, the emergence of clinically resistant pathogenic bacteria seems to be occurring more rapidly.

Biologically, antibiotic resistance is a natural consequence of the interaction between the environmental selective pressure exerted by antibiotic use and the adaptive capacity of pathogenic organisms governed by genetic mutation. Although the large-scale use of antibiotics is undoubtedly a key factor in the development and spread of antibiotic resistance into a problem and crisis, recent research indicates that resistance to antibiotic compounds originally isolated from natural products is a natural phenomenon that existed even before their industrial production. Known mechanisms of resistance generally include: (1) chemical alteration and inactivation of antibiotics, primarily through hydrolysis by β-lactamases; (2) modification of the drug binding site on penicillin-binding proteins (PBPs); (3) reduction of intracellular drug accumulation, e.g., via active efflux pumps; (4) loss or alteration of outer membrane porins in bacteria, restricting drug influx; (5) formation of biofilms by resistant bacteria.

The hydrolytic destruction of β-lactam antibiotics by β-lactamases represents the most clinically relevant resistance mechanism in pathogens. The vast majority of drug-resistant bacteria on the priority or urgent threat lists of the WHO and the US CDC utilize the expression of β-lactamases as their primary mechanism. The target site of β-lactam antibiotics is the penicillin-binding proteins on the bacterial cell membrane, whose biochemical nature is a series of enzymes involved in the biosynthesis and self-degradation of bacterial cell wall peptidoglycan (PG), including DD-transpeptidases (TP), DD-carboxypeptidases, and DD-endopeptidases. The core β-lactam bicyclic nucleus of typical β-lactam antibiotics closely mimics the structure of the D-alanyl-D-alanine (D-ala-D-ala) portion of the peptidoglycan tetrapeptide side chain, the normal substrate of PBPs. Normal levels and types of PBPs are essential for bacteria to maintain normal cell morphology and function, and to complete cell division and morphogenesis during the cell cycle. β-lactam antibiotics effectively interfere with the normal physiological cycle of cell wall biosynthesis and degradation through irreversible covalent binding to PBPs, directly disrupting the bacterial cell wall's protective function against osmotic shock, leading to bacterial cell lysis and death. However, some Gram-negative bacteria and a few Gram-positive species can produce β-lactamases structurally similar to PBPs. These enzymes also possess ultra-high affinity for many β-lactam antibiotics but can hydrolyze and destroy the four-membered β-lactam ring of the bound antibiotic, conferring resistance. The various classes of β-lactamases evolving at an astonishing rate exhibit immense molecular diversity, with over 7,000 different β-lactamase protein molecules known globally today. Many pathogenic bacteria can express multiple β-lactamases with different specificities, and some β-lactamases have evolved extended-spectrum hydrolytic activity (ESBLs, carbapenemases, etc.), thus constituting a pathway to acquired multidrug resistance (MDR, XDR, PDR, etc.).

Addressing this resistance mechanism, a straightforward strategy is to develop small molecule inhibitors of β-lactamases (BLIs) and use them in combination therapy to restore the antibacterial activity of otherwise tolerated β-lactam antibiotics. For example, Patent Publication No. CN104364254B discloses heterobicyclic compounds as β-lactamase inhibitors. When these heterobicyclic compounds are used in combination with traditional β-lactam antibiotics, they can reverse the resistance of most bacteria to β-lactam antibiotics caused by the expression of β-lactamases.

However, the efficacy of the aforementioned β-lactamase inhibitors still has room for further improvement. Therefore, it is necessary to design β-lactamase inhibitors with novel molecular structures.

### SUMMARY

To address the problems of the prior art, the present invention provides a urea-containing tricyclic β-lactamase inhibitor, preparation method therefor and use thereof. The objective is to provide a new class of β-lactamase inhibitors.

The present invention provides a compound of Formula I, or a pharmaceutically acceptable salt, a stereoisomer, or a solvate thereof, 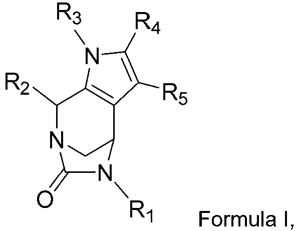 wherein,
R₁ is 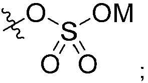
R₂ is selected from the group consisting of hydrogen, halogen, cyano, monofluoromethyl, difluoromethyl, trifluoromethyl, -SO₂C₁₋₆ alkyl, -SO₂C₃₋₆ cycloalkyl, -SO₂ aryl, -SO₂ heteroaryl, -C(O)NR₇R₈, -CH₂NR₇R₈, -C(NH)NR₇R₈, -C(O)OR₉, -C(NH)OR₉, - C(O)NR₁₀NR₇R₈, -C(O)NR₁₀NR₁₁C(O)R₁₂, -C(O)NR₁₀OR₉, -C(NH)NR₁₀OR₉, - C(O)NR₁₀(CH₂)mNR₇R₈, -C(O)NR₁₀(CH₂)mNR₁₁C(O)R₁₂, and -CH₂NR₇C(O)R₉
R₃ is selected from the group consisting of hydrogen, C₁₋₆ alkyl, C₃₋₆ cycloalkyl, C₄₋₇ heterocycloalkyl, C₃₋₆ cycloketone, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and -C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl, heterocycloalkyl, and cycloketone are optionally substituted with zero, one, or more substituents each independently selected from the group consisting of C₁₋₆ alkyl, hydroxyl, halogen, and -NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and - C(O)NR₁₅R₁₆;
wherein the aryl and heteroaryl of R₄ and R₅ are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₃ and R₁₄ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₅ and R₁₆ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
m is 1, 2, 3 or 4; and M is selected from the group consisting of hydrogen, a metal ion, and an organic cation.

Furthermore, R₂ is selected from the group consisting of hydrogen, cyano, - C(O)NR₇R₈, -CH₂NR₇R₈, -C(NH)NR₇R₈, -C(O)OR₉, -C(NH)OR₉, -C(O)NR₁₀NR₇R₈, - C(O)NR₁₀NR₁₁C(O)R₁₂, -C(O)NR₁₀OR₉, -C(NH)NR₁₀OR₉, -C(O)NR₁₀(CH₂)mNR₇R₈, - C(O)NR₁₀(CH₂)mNR₁₁C(O)R₁₂, and -CH₂NR₇C(O)R₉
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl; and m is 1, 2, 3, or 4.

Furthermore, the compound is of Formula I-A: 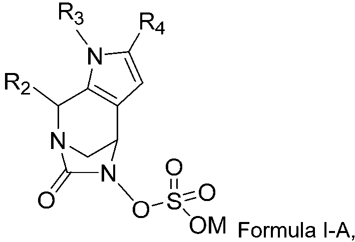 wherein,
R₂ is selected from the group consisting of hydrogen, halogen, cyano, monofluoromethyl, difluoromethyl, trifluoromethyl, -C(O)NR₇R₈, -CH₂NR₇R₈, -C(O)OR₉, and - CH₂NR₇C(O)R₉;
R₃ is selected from the group consisting of hydrogen, C₁₋₆ alkyl, C₃₋₆ cycloalkyl, - C(O)C₁₋₆ alkyl, -C(O)C₃₋₆ cycloalkyl, -C(O)C₂₋₆ alkenyl, -C(O) phenyl, -C(O) pyrazolyl, - C(O)NR₁₃R₁₄, -SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and - NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆;
wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, and piperidinyl;
R₉ is selected from the group consisting of hydrogen and C₁₋₆ alkyl;
R₁₃, R₁₄, R₁₅, and R₁₆ are each independently selected from the group consisting of hydrogen and C₁₋₆ alkyl; and M is selected from the group consisting of hydrogen and a sodium ion.

Furthermore, the compound is of Formula I-B: 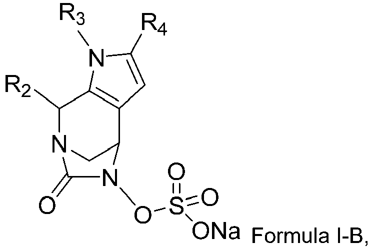 wherein,
R₂ is selected from the group consisting of hydrogen, halogen, cyano, monofluoromethyl, difluoromethyl, trifluoromethyl, -C(O)NR₇R₈, -CH₂NR₇R₈, -C(O)OR₉, and - CH₂NR₇C(O)R₉;
R₃ is selected from the group consisting of hydrogen, C₁₋₆ alkyl, C₃₋₆ cycloalkyl, - C(O)C₁₋₆ alkyl, -C(O)C₃₋₆ cycloalkyl, -C(O)C₂₋₆ alkenyl, -C(O) phenyl, -C(O) pyrazolyl, - C(O)NR₁₃R₁₄, -SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and - NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆;
wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, and piperidinyl;
R₉ is selected from the group consisting of hydrogen and C₁₋₆ alkyl; and R₁₃, R₁₄, R₁₅, and R₁₆ are each independently selected from the group consisting of hydrogen and C₁₋₆ alkyl.

Furthermore, the compound is of Formula I-C: 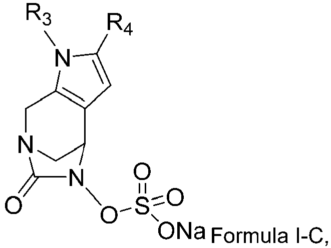 wherein,
R₃ is selected from the group consisting of hydrogen, C₁₋₆ alkyl, C₃₋₆ cycloalkyl, - C(O)C₁₋₆ alkyl, -C(O)C₃₋₆ cycloalkyl, -C(O)C₂₋₆ alkenyl, -C(O) phenyl, -C(O) pyrazolyl, - C(O)NR₁₃R₁₄, -SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and - NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆;
wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆; and R₁₃, R₁₄, R₁₅, and R₁₆ are each independently selected from the group consisting of hydrogen and C₁₋₆ alkyl.

Furthermore, the compound is one of the following compounds:

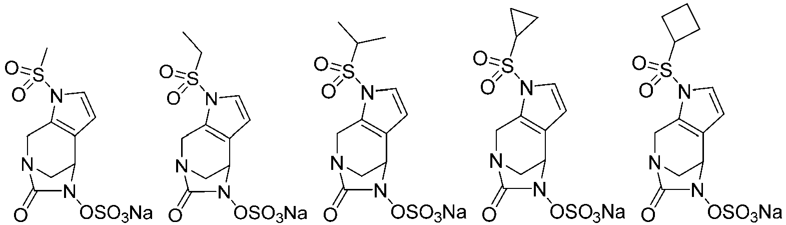

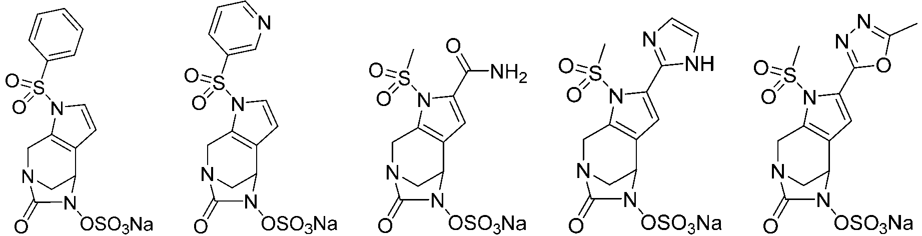

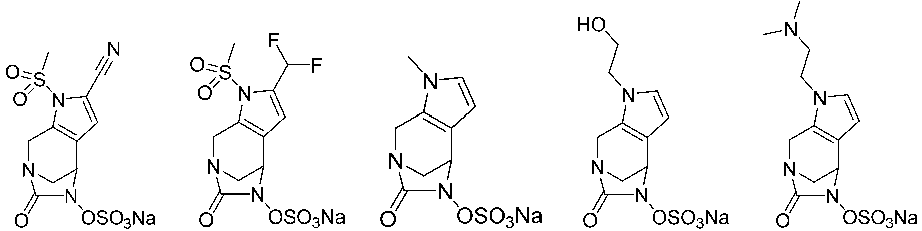

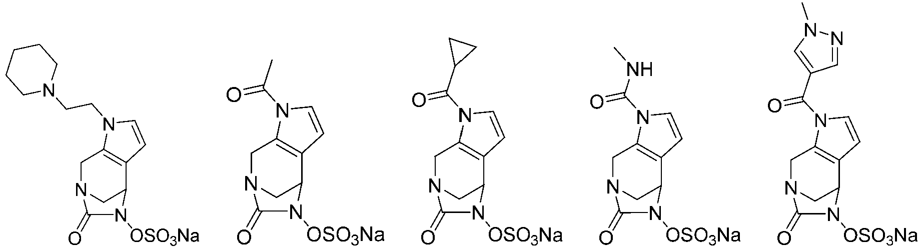

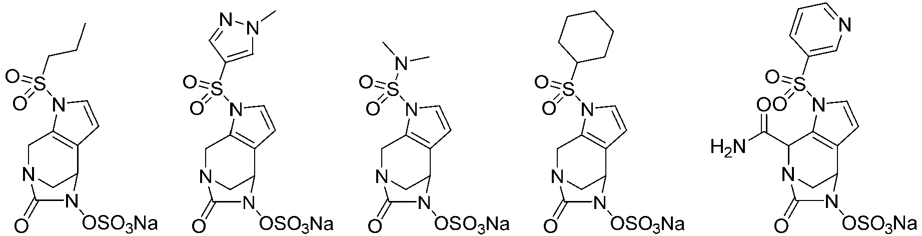

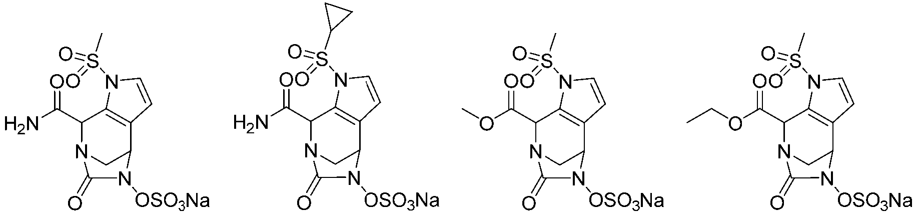

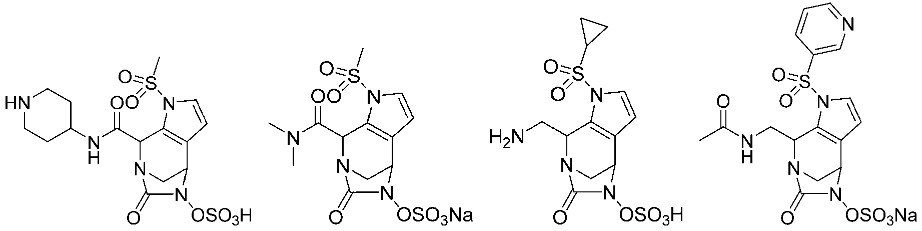

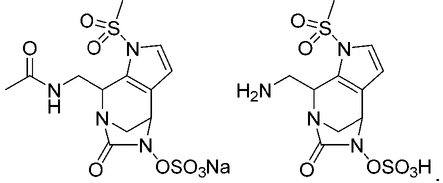

Furthermore, the compound is of Formula II: 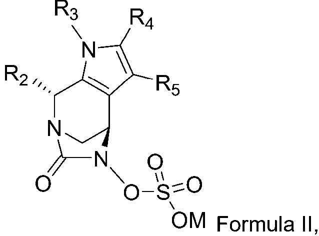 wherein:
R₂ is selected from the group consisting of hydrogen, halogen, cyano, monofluoromethyl, difluoromethyl, trifluoromethyl, -SO₂C₁₋₆ alkyl, -SO₂C₃₋₆ cycloalkyl, -SO₂ aryl, -SO₂ heteroaryl, -C(O)NR₇R₈, -CH₂NR₇R₈, -C(NH)NR₇R₈, -C(O)OR₉, -C(NH)OR₉, - C(O)NR₁₀NR₇R₈, -C(O)NR₁₀NR₁₁C(O)R₁₂, -C(O)NR₁₀OR₉, -C(NH)NR₁₀OR₉, - C(O)NR₁₀(CH₂)mNR₇R₈, -C(O)NR₁₀(CH₂)mNR₁₁C(O)R₁₂, and -CH₂NR₇C(O)R₉
R₃ is selected from the group consisting of hydrogen, C₁₋₆ alkyl, C₃₋₆ cycloalkyl, C₄₋₇ heterocycloalkyl, C₃₋₆ cycloketonyl, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and -C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and wherein the cycloalkyl, heterocycloalkyl, and cycloketonyl are optionally substituted with zero, one, or more substituents each independently selected from the group consisting of C₁₋₆ alkyl, hydroxyl, halogen, and - NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and - C(O)NR₁₅R₁₆; wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₃ and R₁₄ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₅ and R₁₆ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
m is 1, 2, 3, or 4; and M is selected from the group consisting of hydrogen, a metal ion, and an organic cation.

Furthermore, the compound is of Formula III: 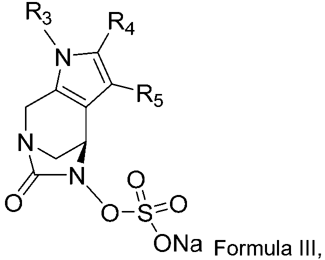 wherein:
R₃ is selected from the group consisting of hydrogen, C₁₋₆ alkyl, C₃₋₆ cycloalkyl, C₄₋₇ heterocycloalkyl, C₃₋₆ cycloketonyl, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and -C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and
wherein the cycloalkyl, heterocycloalkyl, and cycloketonyl are optionally substituted with zero, one, or more substituents each independently selected from the group consisting of C₁₋₆ alkyl, hydroxyl, halogen, and -NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and - C(O)NR₁₅R₁₆; wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₃ and R₁₄ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen; and
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and - C(O) heteroaryl; or R₁₅ and R₁₆ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen.

Furthermore, the compound is of Formula IV

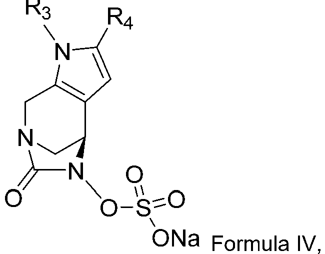

Wherein: R₃ is selected from the group consisting of hydrogen, C₁₋₆ alkyl, C₃₋₆ cycloalkyl, -C(O)C₁₋₆ alkyl, -C(O)C₃₋₆ cycloalkyl, -C(O)C₂₋₆ alkenyl, -C(O) phenyl, -C(O) pyrazolyl, -C(O)NR₁₃R₁₄, -SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄; wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and - C(O)NR₁₃R₁₄; wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and wherein the cycloalkyl is optionally substituted with zero, one, or more substituents each independently selected from the group consisting of C₁₋₆ alkyl, hydroxyl, halogen, and -NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆; wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆; and
R₁₃, R₁₄, R₁₅, and R₁₆ are each independently selected from the group consisting of hydrogen and C₁₋₆ alkyl.

Furthermore, the compound is one of the following compounds:

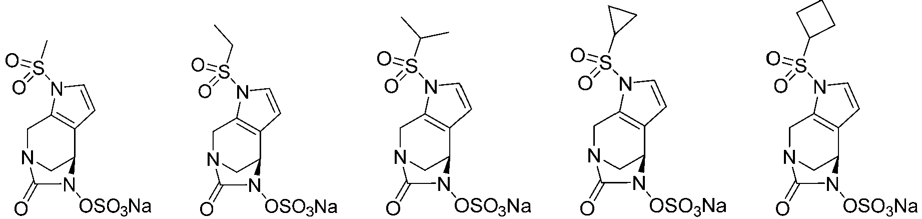

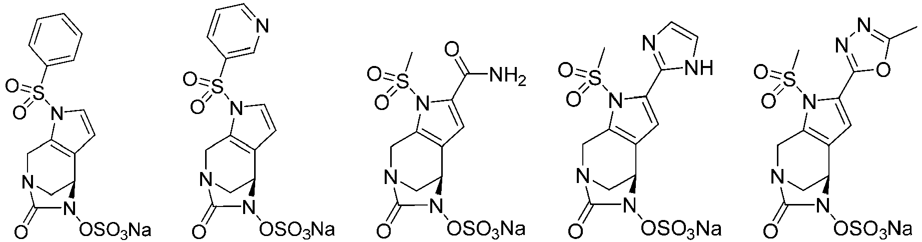

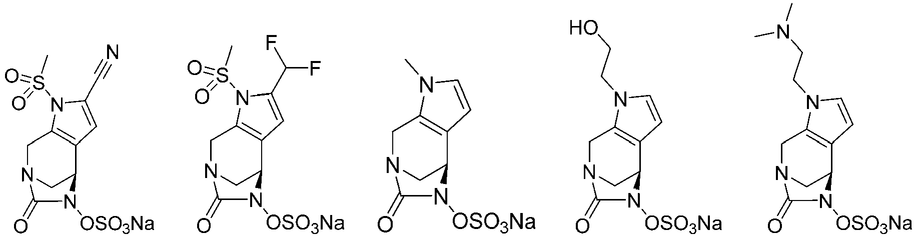

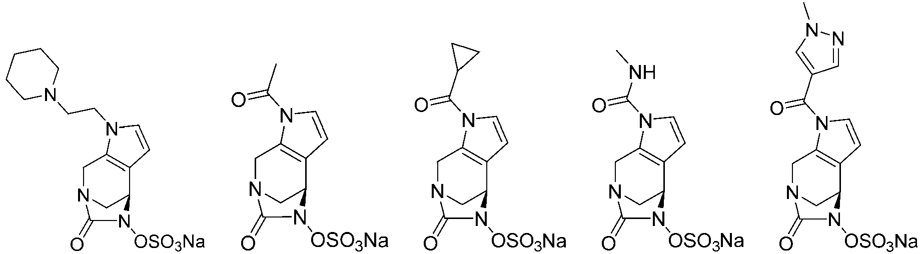

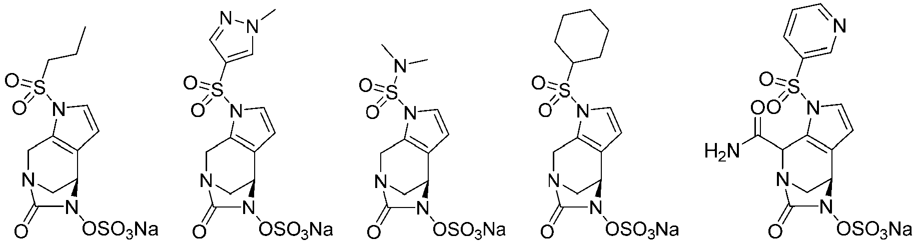

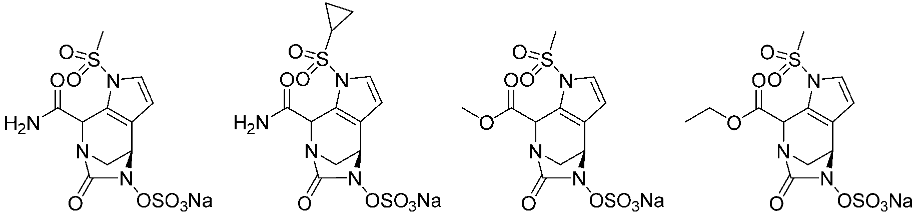

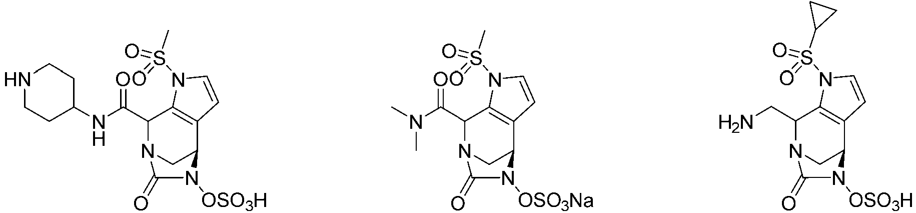

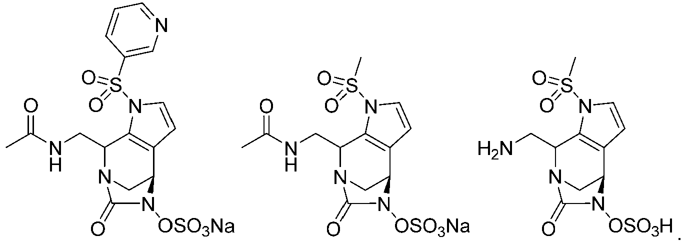

The present invention also provides a method for preparing the aforementioned compound, comprising: 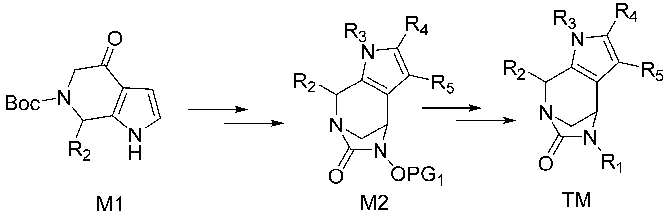
Step 1: subjecting a bicyclic compound M1 to a reduction reaction, a Mitsunobu reaction, a substitution reaction, a deprotection reaction, and a cyclization reaction to obtain a tricyclic intermediate M2;
Step 2: subjecting the tricyclic intermediate M2 to a deprotection reaction, a substitution reaction, and an ion-exchange reaction to obtain a target compound TM;
wherein R₁, R₂, R₃, R₄, and R₅ are as defined above; and
PG₁ is selected from hydroxyl protecting groups.

Furthermore, the preparation method of compound M1 comprises: 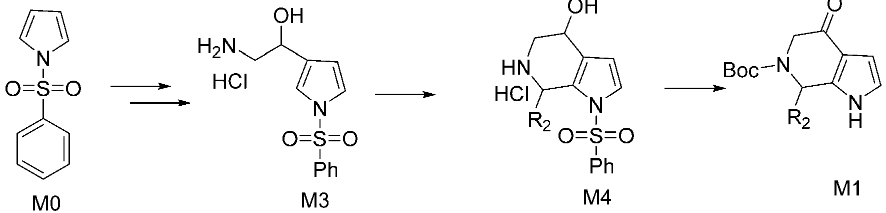 subjecting a compound M0 to a Friedel-Crafts reaction, a substitution reaction, and a reduction reaction to obtain an intermediate M3; cyclizing the intermediate M3 to obtain a bicyclic intermediate M4; and subjecting the bicyclic intermediate M4 to a protection reaction, an oxidation reaction, and a deprotection reaction to obtain the intermediate M1; wherein R₂ is as defined above.

The present invention also provides use of the aforementioned compound, or a pharmaceutically acceptable salt, a stereoisomer, or a solvate thereof, in the preparation of a β-lactamase inhibitor.

The present invention also provides use of the aforementioned compound, or a pharmaceutically acceptable salt, a stereoisomer, or a solvate thereof, in the preparation of a medicament for treating bacterial infection.

Furthermore, the medicament is for treating a drug-resistant bacterial infection expressing β-lactamase.

The present invention also provides use of the aforementioned compound, or a pharmaceutically acceptable salt, a stereoisomer, or a solvate thereof, in the preparation of a medicament for enhancing bacterial sensitivity to antibiotics.

Furthermore, the medicament enhances the sensitivity of β-lactamase-producing drug-resistant bacteria to β-lactam antibiotics.

The present invention also provides a medicament for treating bacterial infection and/or enhancing bacterial sensitivity to antibiotics, wherein the medicament comprises, as an active ingredient, the aforementioned compound, or a pharmaceutically acceptable salt, a stereoisomer, or a solvate thereof, together with one or more pharmaceutically acceptable excipients or auxiliary ingredients.

Furthermore, the medicament is for treating a drug-resistant bacterial infection expressing β-lactamase and/or enhances the sensitivity of β-lactamase-producing drug-resistant bacteria to β-lactam antibiotics.

The present invention also provides a combination medicament for treating bacterial infection, wherein the combination medicament comprises the aforementioned medicament and at least one β-lactam antibiotic.

The present invention also provides a pharmaceutical composition for treating bacterial infection, wherein the pharmaceutical composition comprises, as an active ingredient, the aforementioned combination medicament together with one or more pharmaceutically acceptable excipients or auxiliary ingredients.

The compounds and derivatives provided in the present invention can be named according to the IUPAC (International Union of Pure and Applied Chemistry) or CAS (Chemical Abstracts Service, Columbus, OH) nomenclature systems.

Definitions of terms used in the present invention: Unless otherwise specified, the initial definitions provided for groups or terms herein apply to said groups or terms throughout the entire specification; for terms not specifically defined herein, the meanings that can be given to them by those skilled in the art should be interpreted based on the disclosure content and context.

"Substituted" means that a hydrogen atom in a molecule is replaced by another different atom or molecule. "Substituted" can mean substitution by one group or by at least two groups.

The minimum and maximum carbon atom content in a hydrocarbon group is indicated by a prefix, for example, the prefix C_{a-b} alkyl indicates any alkyl group containing "a" to "b" carbon atoms. Thus, for example, "C₁₋₆ alkyl" refers to an alkyl group containing 1 to 6 carbon atoms.

"Alkyl" refers to a saturated hydrocarbon chain having a specified number of carbon atoms. For example, C₁₋₆ alkyl refers to an alkyl group having 1 to 6 carbon atoms, i.e., 1, 2, 3, 4, 5, or 6 carbon atoms. Alkyl groups may be straight or branched. Representative branched alkyl groups have one, two, or three branches. Alkyl groups may be optionally substituted with one or more substituents as defined herein. Alkyl includes methyl, ethyl, propyl (n-propyl and isopropyl), butyl (n-butyl, isobutyl, and t-butyl), pentyl (n-pentyl, isopentyl, and neopentyl), hexyl, and the like. Alkyl groups may also be part of other groups, such as C₁₋₆ alkoxy.

"Alkenyl" is used to denote a straight or branched hydrocarbon group containing at least one olefinic bond, e.g., C₂₋₁₂ alkenyl refers to a straight or branched hydrocarbon group containing 2 to 12 carbon atoms and at least one olefinic bond.

"Alkynyl" is used to denote a straight or branched hydrocarbon group containing at least one acetylenic bond, e.g., C₂₋₁₂ alkynyl refers to a straight or branched hydrocarbon group containing 2 to 12 carbon atoms and at least one acetylenic bond.

"Cycloalkenyl" refers to a non-aromatic carbocyclic group containing at least one olefinic bond, including cyclized alkenyl. Cycloalkenyl may include bicyclic, polycyclic, and spiro ring systems. Examples of cycloalkenyl include, but are not limited to, cyclopentenyl, cyclohexenyl.

"Alkoxy" is used to denote a saturated chain-like or cyclic hydrocarbon group attached to the substituted atom via an oxygen atom. Examples of alkoxy include, but are not limited to, methoxy, ethoxy, isopropoxy, t-butoxy, cyclopropoxy, cyclobutoxy.

"Alkylthio" is used to denote a saturated chain-like or cyclic hydrocarbon group attached to the substituted atom via a sulfur atom. Examples of alkylthio include, but are not limited to, methylthio, ethylthio, isopropylthio, t-butylthio, cyclopropylthio, cyclobutylthio.

"Cycloalkyl" refers to a saturated or partially saturated, all-carbon monocyclic or polycyclic (including fused, spiro, or bridged) ring group that does not have a conjugated π electron system. For polycyclic systems having aromatic and non-aromatic rings without ring heteroatoms, the term "cycloalkyl" applies when the point of attachment is at a non-aromatic carbon atom (e.g., 5,6,7,8-tetrahydronaphthalen-5-yl). The term "cycloalkyl" includes cycloalkenyl groups, such as cyclohexenyl. Examples of cycloalkyl groups include, for example, adamantyl, cyclopropyl, cyclobutyl, cyclohexyl, cyclopentyl, cyclooctyl, cyclopentenyl, and cyclohexenyl.

"Heterocycloalkyl" refers to a cycloalkyl group wherein at least one carbon atom on the ring is replaced by a heteroatom, the heteroatom being O, N, or S, including, for example, but not limited to:

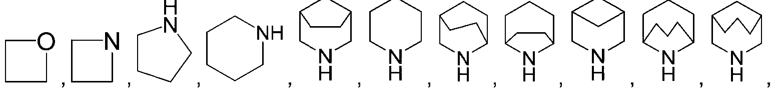

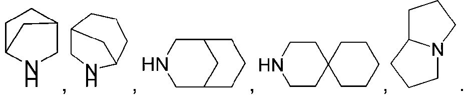

"Aryl" refers to an all-carbon monocyclic or polycyclic (including fused, spiro, or bridged) ring group having a conjugated π electron system, including, for example, but not limited to: phenyl, naphthyl, phenanthryl, anthracenyl, fluorenyl, and indenyl. Said aromatic ring may be fused to other cyclic groups (including saturated and unsaturated rings) but cannot contain heteroatoms such as O, N, or S, and the point of attachment to the parent must be on a carbon atom of the ring having a conjugated π electron system, including, for example, but not limited to

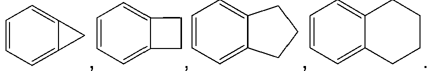

"Heteroaryl" refers to an aryl group wherein at least one carbon atom on the ring of the conjugated π electron system is replaced by a heteroatom, the heteroatom being O, N, or S, including, for example, but not limited to:

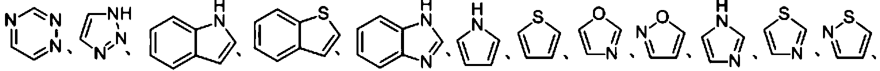

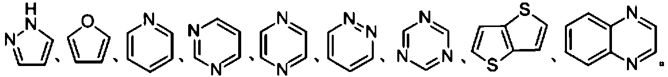

"Cycloketonyl" refers to a cycloalkyl or heterocycloalkyl group wherein at least one carbon is replaced by C=O, including, for example, but not limited to:

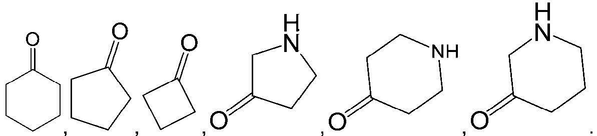

"A saturated ring containing one or two heteroatoms" refers to a heterocycloalkyl group containing one or two heteroatoms.

In the present invention, the structure of -SO₂C₁₋₆ alkyl is 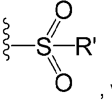 wherein R' is C₁₋₆ alkyl. Similarly, in -SO₂C₃₋₆ cycloalkyl, -SO₂ aryl, -SO₂ heteroaryl, R' is C₃₋₆ cycloalkyl, aryl, heteroaryl, respectively.

In the present invention, the structure of -C(O)C₁₋₆ alkyl is 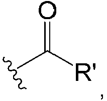 wherein R' is C₁₋₆ alkyl. Similarly, in -C(O)C₃₋₆ cycloalkyl, -C(O)C₂₋₆ alkenyl, -C(O) aryl, -C(O) heteroaryl, R" is C₃₋₆ cycloalkyl, C₂₋₆ alkenyl, aryl, heteroaryl, respectively.

In the present invention, the structure of -C(O)OC₁₋₆ alkyl is 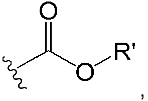 wherein R' is C₁₋₆ alkyl.

In the present invention, the structure of -OC₁₋₆ alkyl is 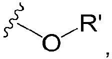 wherein R' is C₁₋₆ alkyl.

In the present invention, the structure of -C(O)NR₇R₈ is 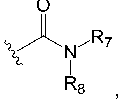 -CH₂NR₇R₈ is 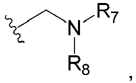 -C(NH)NR₇R₈ is 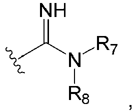 -C(O)OR₉ is 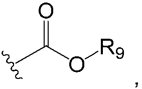 -C(NH)OR₉ is 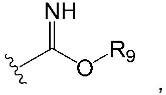 -C(O)NR₁₀NR₇R₈ is 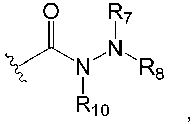 -C(O)NR₁₀NR₁₁C(O)R₁₂ is 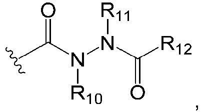 -C(O)NR₁₀OR₉ is 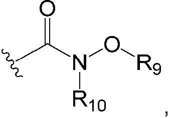 -C(NH)NR₁₀OR₉ is 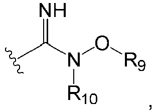 C(O)NR₁₀(CH₂)mNR₇R₈ is 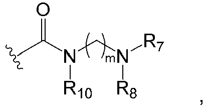 -C(O)NR₁₀(CH₂)MNR₁₁C(O)R₁₂ is 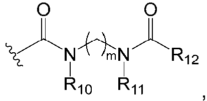 -SO₂NR₇R₈ is 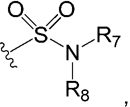 and -NR₇R₈ is

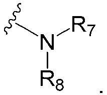

"Stereoisomers" include enantiomers and diastereomers.

The hydrogen atoms in the compounds of the present invention may be various isotopes of hydrogen, for example: protium (¹H), deuterium (²H), or tritium (³H).

The term "pharmaceutically acceptable" means that a carrier, vehicle, diluent, excipient, and/or salt formed is generally chemically or physically compatible with the other ingredients comprising a pharmaceutical dosage form and physiologically compatible with the recipient.

The terms "salt" and "pharmaceutically acceptable salt" refer to acid and/or base salts of the above-mentioned compounds or their stereoisomers with inorganic and/or organic acids and bases, also including zwitterionic salts (inner salts), and also include quaternary ammonium salts, such as alkylammonium salts. These salts may be obtained directly during the final isolation and purification of the compound. They may also be obtained by mixing the above-mentioned compound, or its stereoisomer, with an appropriate amount of acid or base (e.g., an equivalent amount). These salts may precipitate out in solution and be collected by filtration, or be recovered after evaporation of the solvent, or be obtained by freeze-drying after reaction in an aqueous medium. The salts in the present invention may be hydrochlorides, sulfates, citrates, benzenesulfonates, hydrobromides, hydrofluorides, phosphates, acetates, propionates, succinates, oxalates, malates, succinates, fumarates, maleates, tartrates, or trifluoroacetates of the compounds.

In certain embodiments, one or more compounds of the present invention may be used in combination with each other. The compounds of the present invention may also optionally be used in combination with any other active agent for the preparation of a medicament or pharmaceutical composition for modulating cell function or treating diseases. If a combination of compounds is used, these compounds may be administered to the subject simultaneously, separately, or sequentially.

The present invention provides a class of β-lactamase inhibitors with entirely novel structures. When these molecules are used in combination with traditional β-lactam antibiotics, they can reverse the resistance of most bacteria to β-lactam antibiotics caused by the expression of β-lactamases, showing promising application prospects in the clinical treatment of bacterial infections.

Obviously, based on the above content of the present invention, according to common technical knowledge and conventional means in the field, other various forms of modifications, substitutions, or alterations can be made without departing from the basic technical idea of the present invention.

The following specific embodiments in the form of examples provide further detailed description of the above content of the present invention. However, this should not be construed as limiting the scope of the above subject matter of the present invention to the following examples. All technologies implemented based on the above content of the present invention fall within the scope of the present invention.

### DETAILED DESCRIPTION

The raw materials and equipment used in the specific embodiments of the present invention are known products, obtained by purchasing commercially available products.

### Example 1

### Synthetic Route:

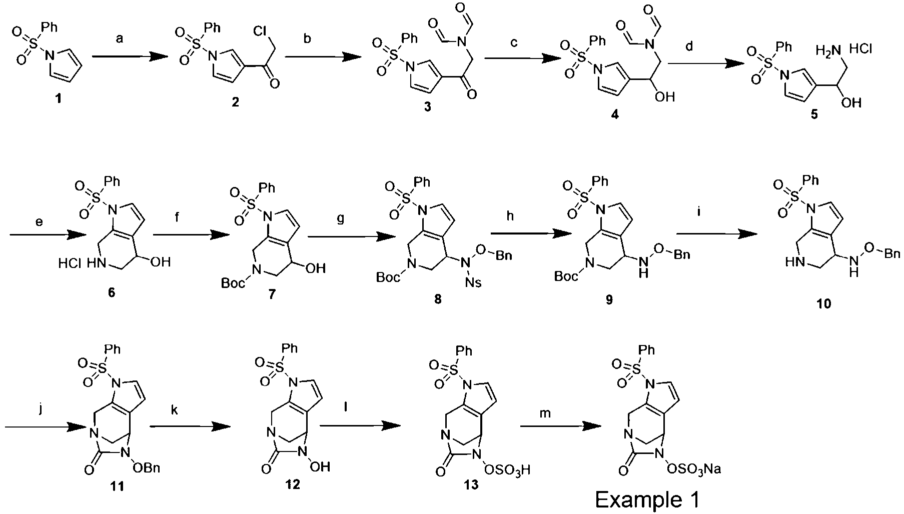

### Synthesis of Intermediate 2 (Step a):

Aluminum chloride (17.69 g, 132.69 mmol) was dissolved in DCM (300 ml). Under nitrogen protection, chloroacetyl chloride (10.56 mL, 132.69 mmol) was added, and the mixture was stirred at room temperature until the reaction solution became a pale-yellow solution. Compound 1 (25 g, 120.63 mmol) was added in one portion, and the mixture was stirred at room temperature under nitrogen protection overnight. The reaction was quenched with 25% wt sodium bicarbonate solution (250 mL) under ice cooling. The layers were separated, and the organic phase was separated. The solvent was evaporated to obtain a crude product, which was purified by column chromatography to give the title compound (25 g, 73%).

### Synthesis of Intermediate 3 (Step b):

Intermediate 2 (11 g, 38.77 mmol) was dissolved in acetonitrile (200 mL). Sodium diformylamide (4.4 g, 46.52 mmol) was added, and the mixture was refluxed overnight. Another batch of sodium diformylamide (2.2 g, 23.26 mmol) was added, and refluxing was continued for 2 h. The acetonitrile was evaporated. The mixture was triturated with EA and filtered. The filter cake was washed with EA (100 mL x 3). The organic phase was dried and concentrated to obtain a crude product, which was purified by column chromatography to give the title compound (9.5 g, 76.5%).

### Synthesis of Intermediate 4 (Step c):

Intermediate 3 (16.62 g, 51.89 mmol) was dissolved in THF/MT=9/1 (200 mL), cooled to -20°C, and stirred for 0.5 h. Ethanol (15 mL, 2.6 mol) was added. Sodium borohydride (2.35 g, 62.27 mmol) was added slowly in portions. After the addition of sodium borohydride was complete, the reaction was continued at -20°C for 2 h, then slowly warmed to 0°C and reacted for 30 min. The reaction was quenched with water under ice cooling to destroy excess sodium borohydride. The solvent was evaporated. The residue was triturated with EA, and inorganic salts were filtered off. The filter cake was washed with EA (100 mL x 3). The organic phase was dried and concentrated to obtain a crude product, which was used directly in the next step.

### Synthesis of Intermediate 5 (Step d):

Intermediate 4 (7 g, 21.72 mmol) was dissolved in HCl/EtOH (1M, 200 mL) and stirred at room temperature overnight. The solvent was evaporated. The residue was triturated with EA and filtered. The filter cake was washed with EA (100 mL x 3) and dried at low temperature. The resulting solid was used directly in the next step.

### Synthesis of Intermediate 6 (Step e):

Intermediate 5 (9.43 g, 35.42 mmol) was dissolved in DMF (150 mL). TFA (7.9 mL, 106.62 mmol) was added in portions at room temperature. Paraformaldehyde (1.5 g, 8.5 mmol) was added, and the mixture was stirred at room temperature overnight. Excess paraformaldehyde was filtered off. The filter cake was washed with DCM (100 mL x 2). The DCM was removed at low temperature, and the residue was used directly in the next step.

### Synthesis of Intermediate 7 (Step f):

TEA (24.62 mL, 177.1 mmol) was added to the DMF solution (150 mL) of intermediate 6, and the mixture was stirred for 30 min. Boc anhydride (12.21 mL, 53.13 mmol) was added, and the mixture was stirred at room temperature overnight. The mixture was diluted with water (100 mL) and extracted with EA (100 mL x 4). The organic phases were combined, washed with water (100 mL x 5), dried, and concentrated to obtain a crude product, which was purified by column chromatography to give the title compound (9.2 g, 70%). 1H NMR (400 MHz, DMSO-d6) δ 7.93 (s, 2H), 7.79 (t, J = 7.4 Hz, 1H), 7.68 (t, J = 7.8 Hz, 2H), 7.33 (d, J = 3.3 Hz, 1H), 6.36 (d, J = 3.4 Hz, 1H), 5.26 (d, J = 5.7 Hz, 1H), 4.52 (s, 2H), 4.40 (d, J = 5.4 Hz, 1H), 3.59 (s, 1H), 3.18 (d, J = 5.3 Hz, 1H), 1.40 (s, 9H).

### Synthesis of Intermediate 8 (Step g):

Intermediate 7 (1 g, 2.64 mmol), NsNHOBn (814 mg, 2.64 mmol), and triphenylphosphine (1.038 g, 3.96 mmol) were dissolved in toluene (30 mL). Under nitrogen protection, DIAD (777 µL, 3.96 mmol) dissolved in toluene (3 mL) was added dropwise under ice cooling. The mixture was stirred at room temperature overnight. The reaction solution was removed to obtain a crude product, which was purified by column chromatography to give the title compound (1.28 g, 73%).

### Synthesis of Intermediate 9 (Step h):

Intermediate 8 (5.3 g, 7.93 mmol) was dissolved in methanol (20 mL). Potassium carbonate (5.48 g, 39.65 mmol) and TGA (1.37 mL, 19.82 mmol) were added, and the mixture was stirred at room temperature overnight. The solvent was evaporated. The residue was dissolved in water (30 mL) and extracted with EA (50 mL x 4). The organic phases were combined, washed with water (20 mL x 3), dried, and concentrated to obtain a crude product, which was purified by column chromatography to give the title compound (2.8 g, 73%).

### Synthesis of Intermediate 10 (Step i):

Under ice cooling, intermediate 9 (2.8 g) was dissolved in HCl/EA (2M, 20 mL) and stirred at room temperature overnight. The reaction solution was neutralized to weak alkalinity with saturated sodium bicarbonate solution under ice cooling. The mixture was extracted with EA (40 mL x 3). The organic phase was removed, and the residue was purified by column chromatography to give the title compound (1.7 g, 70%).

### Synthesis of Intermediate 11 (Step j):

At -25°C, intermediate 10 (314 mg, 0.82 mmol) was dissolved in ACN (72 mL). DIPEA (542 µL, 3.28 mmol) was added, and the mixture was stirred for 30 min. Triphosgene (97 mg, 0.33 mmol) dissolved in acetonitrile (5 mL) was slowly added dropwise to the reaction solution. Stirring was continued at -25°C for 2 h, then the mixture was slowly warmed to room temperature and stirred overnight. The solvent was evaporated, and the residue was purified by column chromatography to give the title compound (235 mg, 70%).

### Synthesis of Intermediate 12 (Step k):

Intermediate 11 (294 mg, 0.72 mmol) was dissolved in methanol (20 mL). Pd/C (121 mg, 1.14 mmol) and palladium hydroxide (121 mg, 0.86 mmol) were added. The atmosphere was replaced with hydrogen three times, and the mixture was stirred at room temperature under a hydrogen atmosphere overnight. The Pd/C was filtered off through diatomaceous earth. The solvent was rapidly evaporated at low temperature, and the residue was used directly in the next step.

### Synthesis of Intermediate 13 (Step I):

Intermediate 12 (100 mg, 0.31 mmol) was dissolved in pyridine (5 mL). Sulfur trioxide pyridine complex (296 mg, 1.86 mmol) was added, and the mixture was stirred at room temperature under nitrogen protection overnight. The mixture was filtered and washed with DCM (10 mL x 3). The organic phase was concentrated, purified and lyophilized by prep-HPLC, to afford the title compound (30 mg, 24%).

### Synthesis of Example 1 Compound (Step m):

Intermediate 13 was dissolved in 5 mL of purified water. Sodium ion exchange resin was added, and the mixture was stirred for 4 h. After filtration, the filter cake was washed successively with water, acetone/water (1/1), and water. The filtrate was lyophilized to obtain the target compound (5 mg, white solid). 1H NMR (400 MHz, DMSO-d6) δ 7.97 -- 7.90 (m, 2H), 7.83 -- 7.74 (m, 1H), 7.70 -- 7.62 (m, 2H), 7.24 (d, J = 3.3 Hz, 1H), 6.33 (d, J = 3.3 Hz, 1H), 4.56 (d, J = 2.7 Hz, 1H), 4.43 (d, J = 16.8 Hz, 1H), 4.32 (d, J = 17.0 Hz, 1H), 2.89 (s, 1H), 2.73 (s, 1H).

### Example 2

### Synthetic Route:

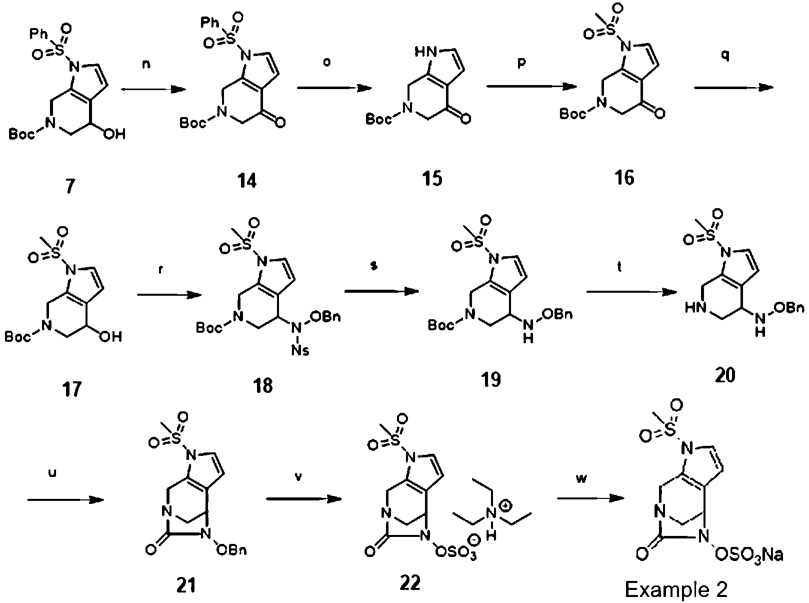

### Synthesis of Intermediate 14 (Step n):

Intermediate 7 (50 mg, 0.13 mmol) was dissolved in EA (8 mL). IBX (73 mg, 0.26 mmol) was added, and the mixture was refluxed overnight. The mixture was filtered. The filter cake was washed with EA (5 mL x 3). The filtrate was evaporated, and the residue was purified by column chromatography to give the title compound (40 mg, 80%).

### Synthesis of Intermediate 15 (Step o):

Intermediate 14 (8 g, 21.25 mmol) was dissolved in ethanol (50 mL). KOH (12 g, 212.5 mmol) was added, and the mixture was stirred at room temperature overnight. The solvent was evaporated. The residue was dissolved in water (50 mL) and extracted with EA (50 mL x 3). The organic phases were combined, concentrated, and purified by column chromatography to give the title compound (4.25 g, 84%).

### Synthesis of Intermediate 16 (Step p):

Intermediate 15 (50 mg, 0.21 mmol) was dissolved in ultra-dry DMF (3 mL). Under nitrogen protection, the mixture was stirred under ice cooling for 10 min. NaH (28 mg) was added, and the mixture was stirred under ice cooling for 30 min. MsCl (50 µL) was added, and the mixture was reacted under ice cooling for 1 h, then warmed to room temperature and reacted for 4 h. The reaction was quenched by adding water under ice cooling to destroy NaH. The mixture was extracted with EA (10 mL x 4). The organic phases were combined, washed with water (20 mL x 5), and evaporated. The residue was purified by column chromatography to give the title compound (60 mg, 90%).

### Synthesis of Intermediate 17 (Step q):

Intermediate 16 (1.5 g, 5.99 mmol) was dissolved in ethanol (20 mL). Sodium borohydride (340 mg, 8.98 mmol) was added in portions under ice cooling. The mixture was slowly warmed to room temperature and reacted at room temperature for 2 h. The reaction was quenched with water to destroy excess sodium borohydride. The solvent was evaporated. The residue was dissolved in EA and washed with water (50 mL x 2). The organic phase was evaporated, and the residue was purified by column chromatography to give the title compound (1 g, 60%).

### Synthesis of Intermediate 18 (Step r):

Intermediate 17 (325 mg, 1.03 mmol), NsNHOBn (318 mg, 1.03 mmol), and triphenylphosphine (540 mg, 2.06 mmol) were dissolved in toluene (15 mL). Under nitrogen protection, DIAD (417 uL, 2.06 mmol) dissolved in toluene (3 mL) was added dropwise under ice cooling. The mixture was stirred at room temperature overnight. The reaction solution was concentrated, and the residue was purified by column chromatography to give the title compound (440 mg, 70%).

### Synthesis of Intermediate 19 (Step s):

Intermediate 18 (440 mg, 0.73 mmol) was dissolved in methanol (8 mL). Potassium carbonate (508 mg, 3.65 mmol) and TGA (127 µl, 1.82 mmol) were added, and the mixture was stirred at room temperature overnight. The solvent was evaporated. The residue was dissolved in water (10 mL) and extracted with EA (10 mL x 4). The organic phases were combined, washed with water (10 mL x 3), dried, concentrated, and purified by column chromatography to give the title compound (200 mg, 65%).

### Synthesis of Intermediate 20 (Step t):

Under ice cooling, intermediate 19 (200 mg, 0.47 mmol) was dissolved in HCl/EA (2M, 7 mL) and stirred at room temperature overnight. The reaction solution was neutralized to weak alkalinity with saturated sodium bicarbonate solution under ice cooling. The mixture was extracted with EA (20 mL x 3). The organic phase was dried, evaporated and concentrated. The residue was purified by column chromatography to give the title compound (143 mg, 94%).

### Synthesis of Intermediate 21 (Step u):

At -25°C, intermediate 20 (143 mg, 0.44 mmol) was dissolved in ACN (40 mL). DIPEA (291 uL, 1.76 mmol) was added, and the mixture was stirred for 30 min. Triphosgene (52 mg, 0.18 mmol) dissolved in acetonitrile (5 mL) was slowly added dropwise to the reaction solution. Stirring was continued at -25°C for 2 h, then the mixture was slowly warmed to room temperature and stirred overnight. The reaction solution was concentrated, and the residue was purified by column chromatography to give the title compound (100 mg, 65%).

### Synthesis of Intermediate 22 (Step v):

Intermediate 21 (100 mg, 0.29 mmol), Pd/C (70 mg, 0.66 mmol), TEA (200 uL, 2.9 mmol), and triethylamine sulfur trioxide complex (120 mg, 0.87 mmol) were dissolved in IPA/EA/H2O=1:2:3 (6 mL). The atmosphere was replaced with hydrogen three times, and the mixture was stirred at room temperature under a hydrogen atmosphere overnight. The reaction was quenched with 1M potassium dihydrogen phosphate solution (2 mL). The Pd/C was filtered off through diatomaceous earth. The filter cake was washed successively with water (10 mL), IPA/water=1:1 (10 mL), and water (10 mL). The aqueous phase was washed with isopropyl acetate (10 mL x 2). Tetrabutylammonium acetate (4 eq) was added to the aqueous phase, and the mixture was stirred at room temperature for 30 min. The mixture was extracted successively with dichloromethane (10 mL x 2) and dichloromethane:isopropanol=5:1 (10 mL x 2). The organic phase was concentrated at 25°C and used directly in the next step.

### Synthesis of Example 2 Compound (Step w):

2 g of Dowex ion exchange resin was suspended in NaOH (2M, 20 mL) solution, stirred slowly for 1 hour, filtered, and packed into a column. The column was washed with a large amount of water until the pH was neutral. At room temperature, the intermediate 22 obtained in the previous step was dissolved in water (5 ml) and added to the above-treated Dowex ion exchange resin. The mixture was stirred slowly for 4 hours, then filtered with suction and washed three times with water (5 mL). The collected fractions were lyophilized to obtain the target compound as a white solid (15 mg, 14.3%). 1H NMR (400 MHz, DMSO-d6) δ 6.98 (d, J = 3.3 Hz, 1H), 6.27 (d, J = 3.3 Hz, 1H), 4.55 (d, J = 2.7 Hz, 1H), 4.46 -- 4.34 (m, 1H), 4.23 (d, J = 16.8 Hz, 1H), 3.40 (dd, J = 10.9, 2.9 Hz, 1H), 3.36 (s, 3H), 3.17 (d, J = 10.9 Hz, 1H).

### General Synthetic Route for Examples 3-12:

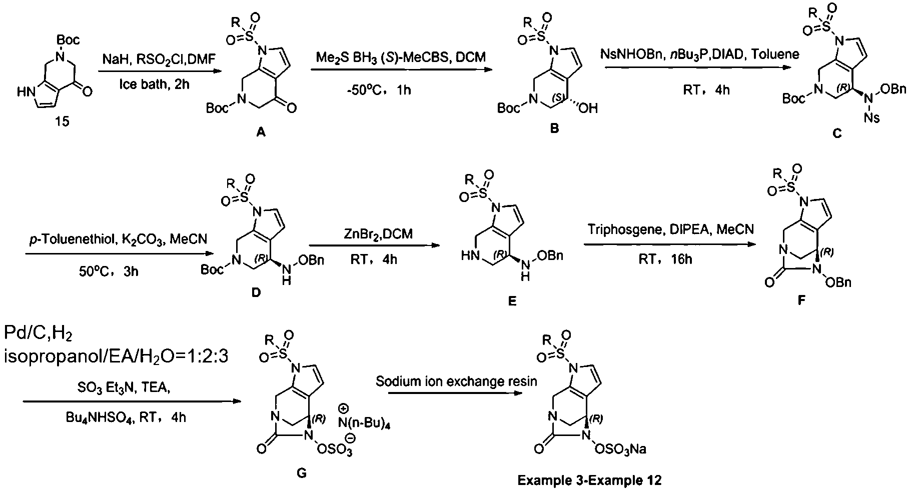

The general synthetic method for Examples 3 to 12 is as described above. Using common intermediate 15 as a starting material, sulfonylation with different sulfonyl chlorides gives intermediate A, followed by asymmetric reduction to obtain the secondary alcohol B. B undergoes a Mitsunobu reaction to obtain the configurationally inverted intermediate C. After two-step deprotection to remove the o-nitrobenzenesulfonyl group and the tert-butoxycarbonyl group, intermediate E is obtained. E undergoes cyclization with triphosgene in acetonitrile to give the tricyclic intermediate F. The latter is debenzylated under Pd/C catalysis and sulfonated to obtain the sulfonic acid tetrabutylammonium salt G. Finally, the sodium salts, Example 3-Example 12, are prepared via sodium ion exchange resin.

The specific preparation method is described below using Example 3 as an example. The preparation methods for Example 4-Example 12 are similar to that of Example 3.

### Example 3

### Synthetic Route:

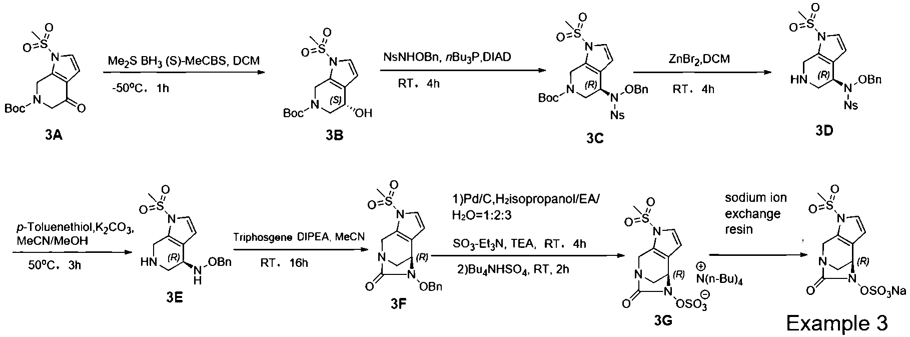

Compound 3A is compound 16 from Example 2, prepared according to the method for compound 16 in Example 2.

### Synthesis of Intermediate 3B

Under nitrogen protection, intermediate 3A (i.e., compound 16, 5.0 g, 15.91 mmol) and (S)-MeCBS (15.91 mL, 2 M, 15.91 mmol) catalyst were dissolved in ultra-dry dichloromethane (50 mL). The temperature was cooled to -50 °C and maintained. Borane dimethyl sulfide complex (7.96 mL, 15.91 mmol) was slowly added dropwise over about 1 hour. After the addition was complete, the mixture was slowly warmed to room temperature and the reaction was continued for about another 1 hour. Isopropanol was added to quench the reaction. The reaction solution was concentrated to obtain a crude product, which was purified by silica gel column chromatography to give compound 3B (4.5 g, 89.4%, enantiomeric excess e.e.% measured by chiral HPLC = 84.4%).

Recrystallization: Heated to 50°C, 4.5 g of the product was dissolved in 10 mL of ethyl acetate. About 50 mL of petroleum ether was added. The mixture was slowly cooled to room temperature, and crystals precipitated. Filtration under reduced pressure gave 2.5 g of pure product, with an enantiomeric excess e.e.% measured by chiral HPLC = 99.1%.

LCMS: Calculated for C₁₃H₁₉N₂O₅S, [M-H]⁻ = 315.1015, found: 315.2.

¹H NMR (400 MHz, CDCl₃) δ = 7.07-7.06 (d, J = 3.2 Hz, 1H), 6.34-6.33 (d, J = 3.6 Hz, 1H), 4.59-4.44 (m, 3H), 3.70 (s, 1H), 3.61-3.58 (d, J = 11.6 Hz, 1H), 3.35 (m, 3H), 1.39 (s, 9H) ppm.

### Synthesis of Intermediate 3C

At room temperature, intermediate 3B obtained in the previous step (2.4 g, 7.59 mmol), NsNHOBn (2.58 g, 8.35 mmol), and tributylphosphine (2.27 mL, 9.11 mmol) were dissolved in toluene (50 mL). At 0 °C, diisopropyl azodicarboxylate (1.80 mL, 9.11 mmol) was slowly added dropwise. After the addition, the mixture was stirred at room temperature for 4 hours. Toluene was removed by distillation under reduced pressure. The residue was concentrated to obtain a crude product, which was purified by silica gel column chromatography to give compound 3C (4.0 g, yield 86.9%, enantiomeric excess e.e.% = 94.3%).

LCMS: Calculated for C₂₆H₃₀N₄O₉S₂Na, [M+Na]⁺ = 629.1352, found: 629.2.

### Synthesis of Intermediate 3D

At room temperature, intermediate 3C obtained in the previous step (250 g, 412.1 mmol) and zinc bromide (464.1 g, 2.06 mol) were dissolved in dichloromethane (5 L) and reacted at room temperature for about 4 hours. After the reaction was complete, about 3 L of water was slowly added to the reaction solution. The organic phase was collected. The aqueous phase was extracted once more with dichloromethane. The organic phases were combined, washed with water and saturated brine, and dried over anhydrous sodium sulfate. The organic phase was distilled under reduced pressure to obtain crude product compound 3D (208 g), which was used directly in the next step.

LCMS: Calculated for C₂₁H₂₂N₄O₇S₂, [M+H]⁺ = 507.09, found: 506.9.

### Synthesis of Intermediate 3E

Intermediate 3D obtained in the previous step (208 g, 410.6 mmol) was dissolved in acetonitrile/methanol = 1/1 solution (2 L). p-Toluenethiol (204.0 g, 1.64 mol) and potassium carbonate (227.0 g, 1.64 mol) were added. The mixture was stirred at 50 °C for about 3 hours. After the reaction was complete, the solvent was removed by concentration. 2 L of water and 2 L of ethyl acetate were added for extraction and washing. The layers were separated, and the organic phase was collected. The aqueous phase was extracted once more with 1 L of ethyl acetate. The organic phases were combined, washed with water and saturated brine, dried over anhydrous sodium sulfate, and concentrated. The obtained crude product was purified by silica gel column chromatography to give compound 3E (100 g, two-step yield 75.8%). The enantioselectivity of the product was maintained at 85-90% ee. LCMS: Calculated for C₁₅H₁₉N₃O₃S, [M+H]⁺ = 322.11, found: 322.0.

### Synthesis of Intermediate 3F

At room temperature, intermediate 3E obtained in the previous step (50 g, 155.6 mmol), diisopropylethylamine (80.4 mL, 622.3 mmol), and acetonitrile (5 L) were mixed. Triphosgene (18.5 g, 62.3 mmol) was dissolved in 1 L of acetonitrile solution. The triphosgene solution was slowly added dropwise to the mixed solution at 0 °C with stirring over about 1 hour. The mixture was warmed back to room temperature and reacted overnight. After the reaction was complete, the solvent was removed by distillation under reduced pressure. The reaction solution was concentrated to obtain a crude product, which was purified by silica gel column chromatography to give compound 3F (35 g, yield 64.7%).

Recrystallization: Heated to 50°C, 70 g of the product was dissolved in 70 mL of ethyl acetate. 20 mL of petroleum ether was added. The mixture was slowly cooled to room temperature, and crystals precipitated to give 33 g (yield 47.1%) of a white solid. The product enantioselectivity was 99.5% ee.

¹H NMR (400 MHz, CDCl₃) δ = 7.43-7.35 (m, 5H), 7.05-7.04 (d, J = 3.2 Hz, 1H), 6.37-6.36 (d, J = 3.6 Hz, 1H), 4.89 (s, 2H), 4.48-4.41 (m, 2H), 4.31-4.27 (d, J = 16.8 Hz, 1H), 3.41 (s, 3H), 3.24-3.21 (d, J = 10.4 Hz, 1H) ppm. LCMS: Calculated for C₁₆H₁₈N₃O₄S, [M+H]⁺ = 348.10, found: 348.1.

### Synthesis of Intermediate 3G

At room temperature, intermediate 3F obtained in the previous step (33.0 g, 94.99 mmol), triethylamine sulfur trioxide complex (51.6 g, 284.97 mmol), palladium on carbon (3 g, 31.35 mmol, 10%), and triethylamine (28.84 g, 284.97 mmol) were dissolved in isopropanol (50 mL), ethyl acetate (100 mL), and water (150 mL). The atmosphere was replaced with hydrogen, and the mixture was reacted at room temperature for about 4 hours. The reaction was monitored; the debenzylation reaction was complete, and the sulfonation reaction was also complete. After the reaction was complete, the solid was removed by filtration under reduced pressure. The aqueous phase was collected by layer separation. The organic phase was extracted once more with 100 mL of water. The aqueous phases were combined and washed once more with 100 mL of ethyl acetate. The aqueous phase was collected. Tetrabutylammonium hydrogen sulfate (322.5 g, 949.9 mmL) was added directly to the aqueous phase, and the mixture was stirred for two hours. This stirred solution was extracted twice with 200 mL of dichloromethane. The organic phases were combined, washed 3 times with water, once with saturated brine, dried over anhydrous sodium sulfate, and concentrated to obtain a crude product, which was purified by column chromatography to give compound 3G (48.4 g, yield 87.8%). The purity of the compound determined by HPLC was 98.6%.

¹H NMR (400 MHz, CDCl₃) δ = 7.05-7.04 (d, J = 3.2 Hz, 1H), 6.34-6.33 (d, J = 3.2 Hz, 1H), 4.62-4.61 (d, J = 2.4 Hz, 1H), 4.47-4.43 (d, J = 16.8 Hz, 1H), 4.32-4.27 (d, J = 16.8 Hz, 1H), 3.48-3.44 (dd, J₁ = 11.2 Hz, J₁ = 3.2 Hz, 1H), 3.42 (s, 3H), 3.25-3.22 (d, J = 11.2 Hz, 1H), 3.12-3.06 (q, J = 7.2, 8H), 1.19-1.16 (t, J = 7.2, 12H) ppm. LCMS: Calculated for C₉H₁₀N₃O₇S₂, [M-H]⁻= 335.9966, found: 336.0.

### Synthesis of Example 3

Intermediate 3G (51 g) was dissolved in 500 mL of purified water. Sodium ion exchange resin was added, and the mixture was stirred for 4 h. After filtration, the filter cake was washed successively with water, acetone/water (1/1), and water. Lyophilization gave the target compound, Example 3 (22.5 g, yield 71.2%, HPLC purity 97.8%).

¹H NMR (400 MHz, CDCl₃) δ = 7.05-7.04 (d, J = 3.2 Hz, 1H), 6.34-6.33 (d, J = 3.2 Hz, 1H), 4.62-4.61 (d, J = 2.4 Hz, 1H), 4.47-4.43 (d, J = 16.8 Hz, 1H), 4.32-4.27 (d, J = 16.8 Hz, 1H), 3.48-3.44 (dd, J₁ = 11.2 Hz, J₁ = 3.2 Hz, 1H), 3.42 (s, 3H), 3.25-3.23 (d, J = 11.2 Hz, 1H) ppm. LCMS: Calculated for C₉H₁₀N₃O₇S₂, [M-H]⁻ = 335.9966, found: 336.0.

### Example 4

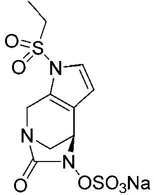

¹H NMR: (400 MHz, DMSO-d6), δ 7.05 (d, J = 2.7 Hz, 1H), 6.36 (d, J = 3.1 Hz, 1H), 4.62 (s, 1H), 4.43 (d, J = 17.0 Hz, 1H), 4.30 (d, J = 16.7 Hz, 2H), 3.56 (q, J = 8.0 Hz, 2H), 3.47 (d, J = 8.2 Hz, 2H), 3.25 (d, J = 10.9 Hz, 2H), 1.09 (t, J = 7.2 Hz, 3H). LCMS: Calculated for C₁₀H₁₂N₃NaO₇S₂, [M-Na]⁻ = 350.3, found: 350.2.

### Example 5

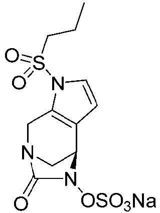

¹H NMR (400 MHz, DMSO-d6) δ 7.05 (d, J = 3.3 Hz, 1H), 6.36 (d, J = 3.2 Hz, 1H), 4.63 (t, J = 2.7 Hz, 1H), 4.44 (d, J = 16.8 Hz, 1H), 4.31 (d, J = 16.9 Hz, 1H), 3.53 (t, 2H), 3.48 (dd, J = 11.0, 2.9 Hz, 1H), 3.26 (d, J = 11.0 Hz, 1H), 1.66 - 1.41 (m, 2H), 0.91 (t, J = 7.4 Hz, 3H). LCMS: Calculated for C₁₁H₁₄N₃O₇S₂⁻Na⁺, [M-Na⁺]⁻ = 364.03, found: 364.0.

### Example 6

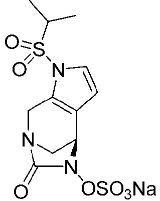

¹H NMR (400 MHz, DMSO-d6) δ 7.05 (d, J = 3.3 Hz, 1H), 6.37 (d, J = 3.3 Hz, 1H), 4.63 (t, J = 2.6 Hz, 1H), 4.40 (d, J = 16.9 Hz, 1H), 4.30 (d, J = 16.8 Hz, 1H), 3.79 - 3.66 (m, 1H), 3.47 (dd, J = 10.9, 2.9 Hz, 1H), 3.26 (d, J = 11.0 Hz, 1H), 1.23 (d, J = 6.8 Hz, 3H), 1.15 (d, J = 6.8 Hz, 3H). LCMS: Calculated for C₁₁H₁₄N₃O₇S₂Na⁺, [M-Na⁺]⁻ = 364.03, found: 364.1.

### Example 7

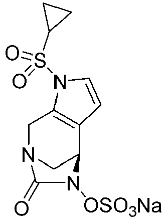

¹H NMR (400 MHz, DMSO-d6) δ 7.08 (d, J = 3.3 Hz, 1H), 6.36 (d, J = 3.3 Hz, 1H), 4.64 (d, J = 2.6 Hz, 1H), 4.46 (d, J = 16.8 Hz, 1H), 4.32 (d, J = 16.7 Hz, 1H), 3.49 (dd, J = 10.9, 2.9 Hz, 1H), 3.26 (d, J = 10.9 Hz, 1H), 3.17 - 3.00 (m, 2H), 1.29 - 1.05 (m, 6H). LCMS: Calculated for C₁₁H₁₂N₃O₇S₂⁻Na⁺, [M-Na⁺]⁻ = 362.01, found: 361.9.

### Example 8

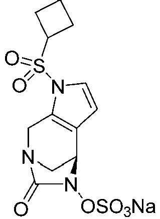

LCMS: Calculated for C₁₂H₁₄N₃O₇S₂, [M-Na]⁻ = 376.03, found: 376.01.

### Example 9

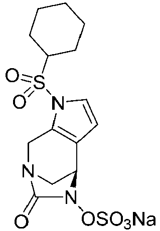

¹H NMR (400 MHz, DMSO-d6) δ 7.01 (d, J = 3.2 Hz, 1H), 6.35 (d, J = 3.2 Hz, 1H), 4.62 (d, J = 2.6 Hz, 1H), 4.43-4.22 (m, 2H), 3.54 (ddd, J = 11.5, 8.0, 3.3 Hz, 1H), 3.47 (dd, J = 11.0, 2.9 Hz, 1H), 3.26 (d, J = 11.0 Hz, 1H), 1.93-1.55 (m, 5H), 1.43-1.03 (m, 5H). LCMS: Calculated for C₁₄H₁₈N₃O₇S₂⁻, [M-Na]⁻ = 404.06, found: 404.03.

### Example 10

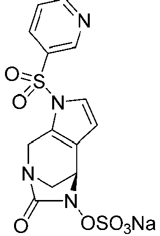

¹H NMR (400 MHz, DMSO) δ 9.13 (d, J = 2.2 Hz, 1H), 8.94 (d, J = 4.8 Hz, 1H), 8.38 (d, J = 8.2 Hz, 1H), 7.71 (dd, J = 8.2, 4.9 Hz, 1H), 7.34 (d, J = 3.2 Hz, 1H), 6.38 (d, J = 3.2 Hz, 1H), 4.58 (d, J = 2.4 Hz, 1H), 4.49 (d, J = 16.9 Hz, 1H), 4.35 (d, J = 16.9 Hz, 1H), 3.45 (d, J = 2.6 Hz, 1H), 3.21 (d, J = 11.0 Hz, 1H). LCMS: Calculated for C₁₃H₁₁N₄O₇S₂, [M-Na]⁻ = 399.37. found 400.1

### Example 11

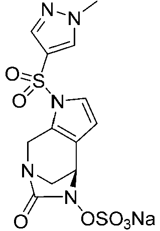

1H NMR (400 MHz, CDCl₃) δ = 8.62 (s, 1H), 8.00 (s, 1H), 7.12-7.11 (d, J = 3.2 Hz, 1H), 6.31-6.30 (d, J = 3.2 Hz, 1H), 4.57-4.57 (d, J = 2.4 Hz, 1H), 4.45-4.32 (q, J = 16.8 Hz, 2H), 3.88 (s, 3H), 3.45-3.42 (m, 1H), 3.21-3.18 (d, J = 10.8, 1H) ppm. LCMS: Calculated for C₁₂H₁₂N₅O₇S₂, [M+H]⁺ = 402.0184, found: 402.2.

### Example 12

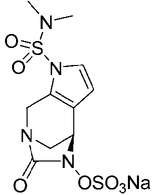

¹H NMR (400 MHz, DMSO-d6) δ 7.08 (d, J = 3.2 Hz, 1H), 6.35 (d, J = 3.2 Hz, 1H), 4.64 (d, J = 2.6 Hz, 1H), 4.46- 4.21 (m, 2H), 3.49 (dd, J = 11.0, 2.9 Hz, 1H), 3.29 (d, J = 10.9 Hz, 1H), 2.77 (s, 6H). LCMS: Calculated for C₁₀H₁₃N₄O₇S₂⁻Na⁺, [M-Na⁺]⁻ = 365.02, found: 364.9.

### General Synthetic Route for Examples 13-15:

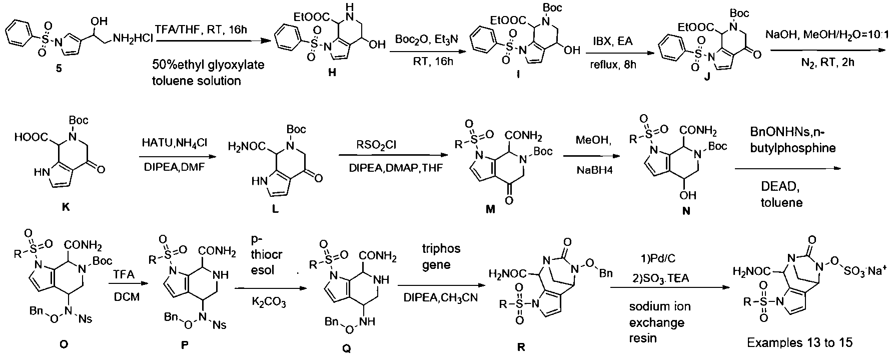

The general synthetic method for Examples 13 to 15 is as described above. Using common intermediate 5 as a starting material, cyclization with ethyl glyoxylate gives intermediate H, followed by Boc protection to obtain intermediate I. I is oxidized with IBX to give ketone J, which undergoes deprotection in a methanol/water solution of sodium hydroxide to obtain key intermediate K. Intermediate K is amidated with ammonium chloride to obtain intermediate L, which is then sulfonylated with different sulfonyl chlorides to obtain different intermediates M. M is then subjected to sodium borohydride reduction, Mitsunobu reaction, and two-step deprotection to obtain intermediate Q. Q undergoes cyclization with triphosgene in acetonitrile to give the tricyclic intermediate R. The latter is debenzylated under Pd/C catalysis and sulfonated to obtain the sulfonic acid tetrabutylammonium salt. Finally, the sodium salts, Example 13-Example 15, are prepared via sodium ion exchange resin.

The specific preparation method is described below using Example 13 as an example. The preparation methods for Example 14-Example 15 are similar to that of Example 13.

### Example 13

### Synthetic Route:

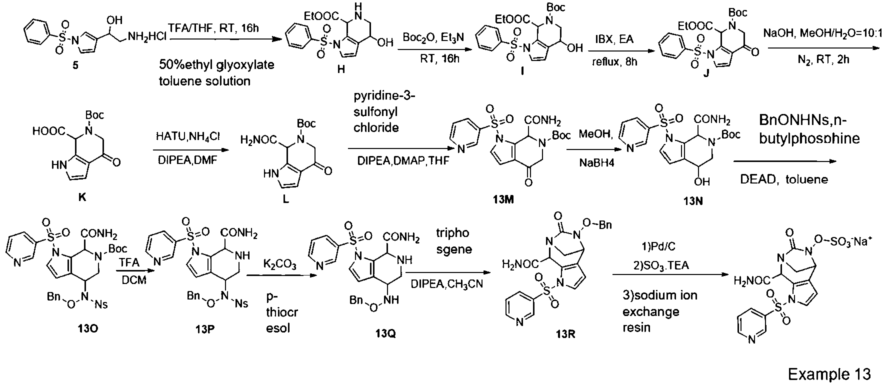

### Synthesis of Intermediate H

Intermediate 5 (230 g, 759.6 mmol), tetrahydrofuran (2.3 L), and 50% ethyl glyoxylate toluene solution (264.5 g, 1.30 mol) were sequentially added to a 10 L reaction flask. The mixture was cooled to 0-5°C in an ice bath. Trifluoroacetic acid (246.2 g, 2.16 mol) was added dropwise. After the addition was complete, the mixture was stirred at room temperature for 16 hours. LCMS monitoring showed no starting material remained and product was formed. After the reaction was complete, the mixture was used directly in the next step.

¹H NMR (400 MHz, DMSO-d6) δ = 1.20-1.26 (t, J = 4.0 Hz, 3H), 2.43-2.49 (m, 1H), 2.93-2.98 (m, 1H), 4.05-4.17 (m, 2H), 4.37-4.39 (m, 1H), 4.71 (s, 1H), 4.96 (s, 1H), 5.18 (s, 1H), 6.41 (d, J = 4.0 Hz, 1H), 7.33 (d, J = 4.0 Hz, 1H), 7.64-7.69 (m, 2H), 7.74-7.78 (m, 1H), 7.87-7.93 (m, 2H). LCMS: C₁₆H₁₈N₂O₅S, Calculated [M] = 350.0, found [M+H]⁺ = 351.1.

### Synthesis of Intermediate I

In a 10 L four-necked flask, the reaction solution containing intermediate H obtained above (estimated as 300 g, 856 mmol) was cooled to 0-5 °C. Triethylamine (434 g, 4.29 mol) was slowly added dropwise. The reaction was warmed to room temperature, then di-tert-butyl dicarbonate (374 g, 1.71 mol) was added dropwise. The mixture was stirred at room temperature for 16 hours. LCMS monitoring showed no starting material remained and product was formed. After the reaction was complete, the mixture was filtered through a pad of diatomaceous earth. The filter cake was washed with ethyl acetate (500 mL x 3) and discarded. The filtrate was concentrated and purified by silica gel column chromatography to obtain 147 g of a yellow solid, yield 38.1%.

¹H NMR (400 MHz, DMSO-d6) δ = 0.96-1.05 (m, 3H), 1.25 (s, 9H), 4.03-4.06 (m, 2H), 4.06-4.08 (m, 1H), 4.24-4.33 (m, 1H), 5.43 (s, 1H), 5.67-5.96 (m, 1H), 6.26-6.29 (m, 1H), 7.22-7.47 (m, 1H), 7.47-7.59 (m, 3H), 7.69-7.74 (m, 2H). LCMS: C₂₁H₂₆N₂O₇S Calculated [M] = 450.1, found [M+H]⁺ = 451.1.

### Synthesis of Intermediate J

Intermediate I (18 g, 39.95 mmol) was added to a 500 mL reaction flask and dissolved with 180 mL of ethyl acetate. 2-lodoxybenzoic acid (22.37 g, 79.9 mmol) was added. The mixture was heated to reflux and reacted for 8 hours. LCMS monitored the reaction until completion. The reaction solution was cooled to room temperature and filtered. The filter cake was washed with 100 mL of ethyl acetate. The filtrate was concentrated and purified by silica gel chromatography column to obtain 15.5 g of a white solid, yield 86%.

¹H NMR (400 MHz, CDCl₃): δ 1.28-1.43 (m, 3H), 1.47-1.62 (m, 9H), 3.64-3.81 (m, 1H), 4.24-4.28 (m, 2H), 4.62-4.82 (m, 1H), 6.54-6.80 (m, 2H), 7.21 (s, 1H), 7.59-7.72 (m, 3H), 7.97-8.02 (m, 2H). LCMS: C₂₁H₂₄N₂O₇S, Calculated [M] = 448.1, found [M+H]⁺ = 449.1.

### Synthesis of Key Intermediate K

Intermediate J (15.5 g, 34.6 mmol), 210 mL of methanol, and 21 mL of water were added to a 500 mL reaction flask. The atmosphere was replaced three times with a nitrogen balloon. The mixture was cooled to 0-5°C in an ice bath. Sodium hydroxide (8.3 g, 207.5 mmol) was added in one portion. The atmosphere was replaced three times with a nitrogen balloon. The mixture was slowly warmed to room temperature and reacted for 2 h. LCMS monitoring showed no starting material remained. The mixture was cooled to 0-5°C in an ice bath, and the pH was adjusted to 1-2 with 2M aqueous hydrochloric acid solution. Methanol was removed by concentration at 40°C. The residue was re-dissolved with 300 mL of ethyl acetate. The aqueous phase was saturated with sodium chloride, stirred evenly, and allowed to stand for layer separation. The aqueous phase was extracted with ethyl acetate (100 mL x 3). The organic phases were combined, dried over anhydrous sodium sulfate, and the organic solvent was evaporated to obtain a total of 10 g of a yellow solid, yield 106%. LCMS: C₁₃H₁₆N₂O₅ ,Calculated [M] = 280.1, found [M+H]⁺ = 281.0.

### Synthesis of Intermediate L:

Intermediate K (10 g, 35.68 mmol), DMF (120 mL), HATU (27.13 g, 71.36 mmol), and ammonium chloride (5.73 g, 107.04 mmol) were added to a 500 mL reaction flask. The atmosphere was replaced three times with a nitrogen balloon. The mixture was cooled to 0-5°C in an ice bath. N,N-Diisopropylethylamine (23.59 g, 142.72 mmol) was added dropwise. After the addition was complete, the mixture was warmed to room temperature and stirred overnight. LCMS monitoring showed no starting material remained. 150 mL of saturated brine, 150 mL of water, and 300 mL of ethyl acetate were added to the reaction solution with stirring. After stirring evenly, the mixture was allowed to stand for layer separation. The aqueous phase was extracted with ethyl acetate (100 mL x 3). The organic phases were combined, dried over anhydrous sodium sulfate, concentrated, and purified by silica gel chromatography column to obtain a total of 7.1 g of the target product, yield 71%.

¹H NMR (400 MHz, DMSO-d6): δ 1.43 (s, 9H), 4.13-4.30 (m, 2H), 5.60-5.68 (m, 1H), 6.35 (s, 1H), 6.95 (s, 1H), 7.41-7.45 (d, J = 16.0 Hz, 1H), 7.54-7.56 (d, J = 8.0 Hz, 1H), 11.38-11.51 (d, J = 12.0 Hz, 1H). LCMS: C₁₃H₁₇N₃O₄, Calculated [M] = 279.1, found [M+H]⁺ = 280.2.

### Synthesis of Intermediate 13M:

Intermediate L (7 g, 25.06 mmol), THF (70 mL), N,N-diisopropylethylamine (9.72 g, 75.18 mmol), N,N-dimethylaminopyridine (0.31 g, 2.51 mmol), and pyridine-3-sulfonyl chloride (6.68 g, 37.59 mmol) were sequentially added to a 250 mL reaction flask. The mixture was stirred at room temperature overnight. LCMS monitored the reaction until no starting material remained. 200 mL of water and 200 mL of ethyl acetate were added to the reaction solution. After stirring evenly, the layers were separated. The organic phase was dried over anhydrous sodium sulfate, concentrated, and purified by silica gel chromatography column to obtain a total of 7.9 g of the target product as a yellow solid, yield 75%.

¹H NMR (400 MHz, DMSO-d6): δ 1.44 (s, 9H), 3.73 (br, 1H), 4.44 (br, 1H), 6.43 (br, 1H), 6.71 (s, 1H), 7.67-7.79 (m, 4H), 8.50-8.53 (m, 1H), 8.97-8.99 (m, 1H), 9.26 (s, 1H). LCMS: C₁₈H₂₀N₄O₆S, Calculated [M] = 420.1, found [M+H]⁺ = 421.1.

### Synthesis of Intermediate 13N:

Intermediate 13M (8 g, 19.03 mmol), THF (100 mL), and methanol (10 mL) were added to a 250 mL reaction flask. The mixture was cooled to 0-5°C in an ice bath. Sodium borohydride (0.86 g, 22.84 mmol) was added in two portions. After the addition was complete, the reaction was continued for 0.5 h. TLC monitored the reaction until no starting material remained. 30 mL of water was added to the reaction solution to quench the reaction. Methanol was removed by concentration. 200 mL of ethyl acetate was added, and the aqueous phase was saturated with sodium chloride. After stirring evenly, the mixture was allowed to stand for layer separation. The organic phase was dried over anhydrous sodium sulfate and concentrated to obtain a total of 8 g of crude target product as a yellow solid, yield 99%.

LCMS: C₁₈H₂₂N₄O₆S, Calculated [M] = 422.1, found [M-H₂O+H]⁺ = 405.1.

### Synthesis of Intermediate 13O:

Intermediate 13N (7.9 g, 18.7 mmol), toluene (120 mL), BnONHNs (11.53 g, 37.4 mmol), and tri-n-butylphosphine (7.57 g, 37.4 mmol) were added to a 250 mL reaction flask. The mixture was cooled to 0-5°C in an ice bath. DEAD (6.51 g, 37.4 mmol) was added dropwise. The mixture was reacted at room temperature overnight. LCMS monitored the reaction until no starting material remained. The reaction solution was concentrated and purified by silica gel chromatography column to obtain a total of 12.3 g of the target product as an orange oil, yield 92%.

¹H NMR (400MHz, DMSO-d6) δ = 1.25-1.50 (m, 9H), 4.03-4.08 (m, 4H), 4.88-4.89 (m, 1H), 5.99-6.00 (m, 1H), 6.59-6.64 (m, 2H), 7.15-7.34 (m, 6H), 7.92-8.45 (m, 5H), 8.83-9.24 (m, 2H). LCMS: C₃₁H₃₂N₆O₁₀S₂, Calculated [M] = 712.2, found [M+H]⁺ = 713.4.

### Synthesis of Intermediate 13P:

Intermediate 130 (11.8 g, 16.56 mmol) was added to a 250 mL reaction flask, followed by a solution of TFA (20 mL) in DCM (100 mL). The mixture was reacted at room temperature for 1 h. LCMS monitored the reaction until no starting material remained. The reaction solution was concentrated. 100 mL of saturated sodium bicarbonate and 200 mL of ethyl acetate were added. After stirring evenly, the mixture was allowed to stand for layer separation. The organic phase was washed with 50 mL of saturated brine, dried over anhydrous sodium sulfate, and concentrated to obtain a total of 11.4 g of crude target product as a yellow oil. Yield 112%.

¹H NMR (400MHz, DMSO-d6) δ 1.25-1.50 (m, 9H), 4.03-4.08 (m, 4H), 4.88-4.89 (m, 1H), 5.99-6.00 (m, 1H), 6.59-6.64 (m, 2H), 7.15-7.34 (m, 6H), 7.92-8.45 (m, 5H), 8.83-9.24 (m, 2H). LCMS: C₃₁H₃₂N₆O₁₀S₂ , Calculated [M] = 712.2, found [M+H]⁺ = 713.4.

### Synthesis of Intermediate 13Q:

Intermediate 130 (11.4 g, 18.61 mmol), p-toluenethiol (6.93 g, 55.83 mmol), and potassium carbonate (7.72 g, 55.83 mmol) were added to a 250 mL reaction flask. The mixture was reacted at room temperature for 3 h. LCMS monitored the reaction until no starting material remained. The reaction solution was concentrated and purified by silica gel chromatography column to obtain a total of 3 g of the target product as a yellow oil, yield 37%.

¹H NMR (400MHz, DMSO-d6) δ 2.62-2.68 (m, 1H), 2.95 (m, 1H), 3.06-3.09 (m, 1H), 3.60-3.62 (m, 1H), 4.61 (s, 2H), 4.66-4.69 (m, 1H), 6.33 (s, 1H), 6.51 (d, J = 8.0 Hz, 1H), 7.28 (s, 1H), 7.30-7.44 (m, 7H), 7.62-7.66 (d, J = 8.0 Hz, 1H), 8.33-8.36 (dd, J = 12.0, 4.0 Hz, 1H), 8.85-8.87 (dd, J = 12.0, 4.0 Hz, 1H), 9.09 (s, 1H). LCMS: C₂₀H₂₁N₅O₄S, Calculated [M] = 427.2, found [M+H]⁺ = 428.2.

### Synthesis of Intermediate 13R:

Intermediate 13Q (3 g, 7.02 mmol), acetonitrile (200 mL), and N,N-diisopropylethylamine (3.63 g, 28.08 mmol) were added to a 250 mL reaction flask. The mixture was cooled to 0-5°C in an ice bath. A solution of triphosgene in acetonitrile (0.83 g triphosgene dissolved in 50 mL acetonitrile) was added dropwise. After the addition was complete, the mixture was reacted at room temperature overnight. LCMS monitored the reaction until no starting material remained. The reaction solution was concentrated and purified by silica gel chromatography column to obtain a total of 2.9 g of the target product as a light yellow solid, yield 91%.

¹H NMR (400MHz, DMSO-d6) δ = 3.16-3.20 (m, 1H), 3.46-3.48 (m, 1H), 4.43 (s, 1H), 4.89 (s, 2H), 5.17 (s, 1H), 6.46 (s, 1H), 7.37-7.68 (m, 7H), 7.68-7.70 (m, 1H), 8.11 (s, 1H), 8.36-8.39 (m, 1H), 8.90-8.92 (m, 1H), 9.11 (s, 1H). LCMS: C₂₁H₁₉N₅O₅S, Calculated [M] = 453.2, found [M+H]⁺ = 454.2.

### Synthesis of Example 13:

Intermediate 13R (518 mg, 1.14 mmol), isopropanol (12 mL), and water (8 mL) were added to a 50 mL reaction flask. Triethylamine (58 mg, 0.57 mmol), triethylamine sulfur trioxide complex (52 mg, 2.85 mmol), and palladium on carbon (1 g, 9.4 mmol) were added sequentially. The atmosphere was replaced three times with hydrogen protection. The mixture was reacted at room temperature overnight. LCMS monitoring showed about 16% of starting material remained. Workup was performed: 1. The reaction solution was filtered with suction through diatomaceous earth. The filter cake was washed with 10 mL of isopropanol. The filtrate was concentrated to dryness at 40°C to obtain a crude product. 2. The crude product was purified by reverse phase column chromatography to obtain a solution of the target product in water and acetonitrile. Acetonitrile was removed by concentration at 40°C. 3. 4 g of Dowex 50WX8 resin was weighed, added to 20 mL of 2M aqueous sodium hydroxide solution, stirred at room temperature for 1 h, and filtered. The filter cake was washed with deionized water until the pH was neutral. The filter cake was transferred to the aqueous solution of the target product and stirred at room temperature for 1 day. 4. The mixture was filtered, and the filtrate was lyophilized. Preparative isolation yielded a total of 51 mg of the sodium salt of the target product as a white solid.

¹H NMR (400MHz, DMSO-d6) δ = 3.26-3.29 (m, 1H), 3.38-3.51 (m, 1H), 4.61 (s, 1H), 5.17 (s, 1H), 6.40 (s, 1H), 7.42-7.46 (m, 2H), 7.66-7.69 (dd, J = 8.0, 4.0 Hz, 1H), 8.11 (s, 1H), 8.36 (d, J = 8.0 Hz, 1H), 8.90 (m, 1H), 9.10 (s, 1H). LCMS: C₁₄H₁₃N₅O₈S₂, Calculated [M] = 443.1, found [M-H]⁻ = 442.0. HPLC: purity 94.9%.

### Example 14

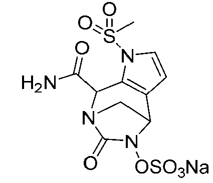

¹H NMR (DMSO-d6, 400MHz): δ 3.29-3.32 (m, 2H), 3.47 (s, 3H), 4.64 (d, J = 4.0 Hz, 1H), 5.08 (s, 1H), 6.36 (d, J = 4.0 Hz, 1H), 7.14 (d, J = 4.0 Hz, 1H), 7.53 (s, 1H), 8.05 (s, 1H). LCMS: Calculated for C₁₀H₁₁N₄NaO₈S₂, [M-Na]⁻ = 379.0, found 379.0.

### Example 15

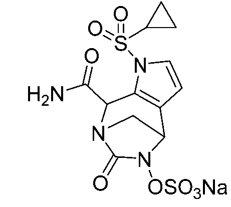

¹H NMR (400MHz, DMSO-d6) δ 1.08-1.27 (m, 5H), 3.20-3.23 (m, 1H), 3.37-3.40 (m, 1H), 4.64 (s, 1H), 5.10 (s, 1H), 6.34 (d, J = 4.0 Hz, 1H), 7.13 (d, J = 4.0 Hz, 1H), 7.47 (s, 1H), 8.04 (s, 1H). LCMS: Calculated for C₁₂H₁₃N₄O₈S₂, [M-Na]⁻ 405.02, found 405.0.

### Example 16

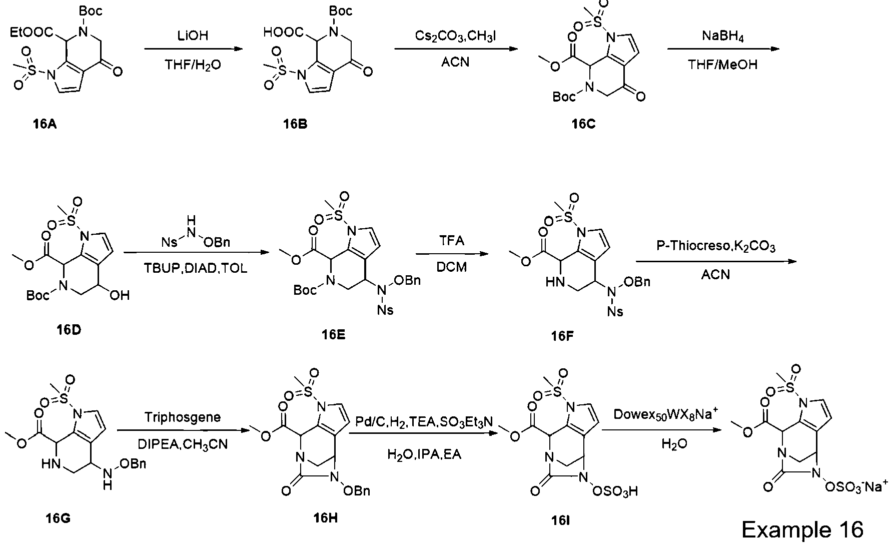

### Synthesis of Intermediate 16B

Intermediate 16A (15 g, 86.43 mmol) was added to a three-necked round-bottom flask and dissolved in tetrahydrofuran (300 mL). The mixture was cooled in an ice bath for 15 minutes. Separately, lithium hydroxide monohydrate (2.12 g, 50.46 mmol) was dissolved in tetrahydrofuran (100 mL) and cooled in an ice bath for 15 minutes. While maintaining the ice bath conditions, the aqueous lithium hydroxide solution was added to the reaction solution. The reaction solution was then slowly warmed to room temperature and stirred for 1 hour. TLC monitored the reaction until completion. Under ice bath conditions, 1M dilute hydrochloric acid was slowly added to the reaction solution to adjust the pH to 2-3. Ethyl acetate (500 mL) was added, and a small amount of solid sodium chloride was added until solid precipitated from the aqueous phase. The reaction solution was allowed to stand, then separated. The aqueous phase was extracted with ethyl acetate (100 mL × 3). The organic phases were combined, dried over anhydrous sodium sulfate, and concentrated to obtain crude intermediate 16B (14 g, yield 100%) as a yellow solid, which was used directly in the next step.

### Synthesis of Intermediate 16C

Compound 16B (6 g, 16.74 mmol) was added to a round-bottom flask, dissolved in acetonitrile (60 mL), and cesium carbonate (8.18 g, 25.11 mmol) was added. The mixture was stirred at room temperature for 5 minutes. Then, iodomethane (4.75 g, 33.4 mmol) was added dropwise using a dropper. The mixture was stirred at room temperature for 6 hours. LCMS monitoring showed no starting material remained.

At room temperature, the reaction solution was slowly added to water (200 mL) and stirred for 10 minutes. Ethyl acetate (150 mL) was added, and stirring was continued for 5 minutes. The reaction solution was allowed to stand, then separated. The aqueous phase was extracted with ethyl acetate (100 mL × 2). The organic phases were combined, washed with saturated brine (200 mL), dried over anhydrous sodium sulfate, and concentrated to obtain a crude yellow oil. Purification by silica gel chromatography column gave intermediate 16C (4.17 g, yield 66.8%) as a yellow oil.

¹H NMR (400MHz, DMSO-d6) δ 1.46 (s, 9H), 3.71 (s, 3H), 3.76-3.88 (m, 3H), 3.93-3.94 (m, 1H), 4.42-4.49 (m, 1H), 4.94 (s, 1H), 6.72 (d, J = 4.0 Hz, 1H), 7.53 (d, J = 4.0 Hz, 1H).

### Synthesis of Intermediate 16D

Intermediate 16C (8 g, 21.48 mmol), tetrahydrofuran (80 mL), and methanol (8 mL) were added to a round-bottom flask. The mixture was cooled to 0-5 °C in an ice bath. Sodium borohydride (1 g, 26.42 mmol) was added in two portions. After the addition was complete, the reaction was continued for 0.5 hours. TLC monitored the reaction until no starting material remained. Water (100 mL) was added to the reaction solution to quench the reaction. Methanol was removed by concentration. Ethyl acetate (300 mL) was added, and the aqueous phase was saturated with sodium chloride. After stirring evenly, the mixture was allowed to stand for layer separation. The organic phase was dried over anhydrous sodium sulfate and concentrated to obtain crude intermediate 16D (8 g, yield 99%) as a white solid, which was used directly in the next step.

### Synthesis of Intermediate 16E

Intermediate 16D (8 g, 21.36 mmol), toluene (100 mL), BnONHNs (12.5 g, 40.54 mmol), and tri-n-butylphosphine (8.2 g, 40.54 mmol) were added to a round-bottom flask. The mixture was cooled to 0-5 °C in an ice bath. DIAD (8.2 g, 40.54 mmol) was added dropwise. The mixture was reacted at room temperature overnight. LCMS monitored the reaction until no starting material remained. The reaction solution was concentrated and purified by silica gel chromatography column to obtain intermediate 16E (10 g, yield 82.9%) as a yellow oil.

### Synthesis of Intermediate 16F

Intermediate 16E (4.6 g, 6.92 mmol) and a solution of TFA in dichloromethane (12.5 mL TFA dissolved in 50 mL dichloromethane) were added to a round-bottom flask. The mixture was reacted at room temperature for 1 hour. TLC monitored the reaction until no starting material remained. The reaction solution was concentrated, and dichloromethane (50 mL) was used to co-evaporate repeatedly 3 times. Saturated sodium bicarbonate solution (100 mL) and ethyl acetate (200 mL) were added. After stirring evenly, the mixture was allowed to stand for layer separation. The aqueous phase was extracted with ethyl acetate (50 mL × 3). The organic phases were combined, dried over anhydrous sodium sulfate, and concentrated to obtain crude intermediate 16F (3.76 g, yield 96.23%) as a yellow solid, which was used directly in the next step.

LCMS: C₂₃H₂₅N₄O₉S₂ ,Calculated [M+H]⁺ = 565.1, found [M+H]⁺ = 565.0.

### Synthesis of Intermediate 16G

Intermediate 16F (3.76 g, 6.66 mmol), acetonitrile (56 mL), p-toluenethiol (2.48 g, 19.98 mmol), and potassium carbonate (2.76 g, 19.98 mmol) were added to a round-bottom flask. The mixture was reacted at room temperature for 3 hours. LCMS monitored the reaction until no starting material remained. The reaction solution was concentrated and purified by silica gel chromatography column to obtain intermediate 16G (1.48 g, yield 58.57%) as a yellow solid.

LCMS: C₁₇H₂₂N₃O₅S ,Calculated [M+H]⁺ = 380.1, found [M+H]⁺ = 380.1.

### Synthesis of Intermediate 16H

Intermediate 16G (1.48 g, 3.90 mmol), acetonitrile (150 mL), and N,N-diisopropylethylamine (2.02 g, 15.6 mmol) were added to a round-bottom flask. The mixture was cooled to 0-5 °C in an ice bath. A solution of triphosgene in acetonitrile (0.46 g, 1.56 mmol, dissolved in 74 mL acetonitrile) was added dropwise. After the addition was complete, the mixture was reacted at room temperature overnight. LCMS monitored the reaction until no starting material remained. The reaction solution was concentrated and purified by silica gel chromatography column to obtain intermediate 16H (1.45 g, yield 91.69%) as a yellow solid.

¹H NMR (400MHz, DMSO-d6) δ 3.25-3.31 (m, 2H), 3.42 (s, 3H), 3.79 (s, 3H), 4.55 (d, J = 4.0 Hz, 1H), 4.93-4.94 (m, 2H), 5.10 (s, 1H), 6.50 (d, J = 4.0 Hz, 1H), 7.26 (d, J = 4.0 Hz, 1H), 7.39-7.46 (m, 5H).

LCMS: C₁₈H₂₀N₃O₆S ,Calculated [M+H]⁺ = 406.1, found [M+H]⁺ = 406.1.

### Synthesis of Intermediate 16I

Intermediate 16H (300 mg, 0.74 mmol), isopropanol (4 mL), ethyl acetate (2 mL), and water (2 mL) were added to a round-bottom flask. Triethylamine (220 mg, 2.22 mmol), triethylamine sulfur trioxide complex (400 mg, 2.22 mmol), and palladium on carbon (60 mg, 0.56 mmol) were added sequentially. The atmosphere was replaced three times with hydrogen protection. The mixture was reacted at room temperature overnight. LCMS monitored the reaction until no starting material remained.

The reaction solution was filtered with suction through diatomaceous earth. The filter cake was washed with ethyl acetate (20 mL). The filtrate was concentrated to dryness below 30°C to obtain a crude product. The crude product was purified by reverse phase preparative chromatography to obtain a solution of the target product in water and acetonitrile. Acetonitrile was removed by concentration below 30°C, and the aqueous solution of intermediate 16I was retained for direct use in the next step.

### Synthesis of Example 16

Dowex 50WX8 resin (10 g) was weighed, added to 2M aqueous sodium hydroxide solution (200 mL), stirred at room temperature for 30 minutes, and filtered. The filter cake was washed with deionized water until the pH was neutral. The filter cake was transferred to the aqueous solution of intermediate 16I and stirred at room temperature for 4 hours. The reaction solution was then filtered, and the filtrate was lyophilized to obtain Example 16 (40 mg, total yield over two steps 13.67%) as a light yellow solid.

¹H NMR (400MHz, DMSO-d6) δ 3.27-3.30 (m, 2H), 3.42 (s, 3H), 3.79 (s, 3H), 4.72 (d, J = 4.0 Hz, 1H), 5.09 (s, 1H), 6.45 (d, J = 4.0 Hz, 1H), 7.25 (d, J = 4.0 Hz, 1H). 13C NMR (150 MHz, DMSO-d6) δ 169.28, 166.77, 125.25, 121.81, 121.10, 112.24, 62.36, 57.80, 53.04, 47.03, 42.10. HRMS (ESI)⁻: calcd for C₁₁H₁₁N₃O₉S₂ [M-Na]⁻: 394.0020, found: 394.0003.

### Example 17

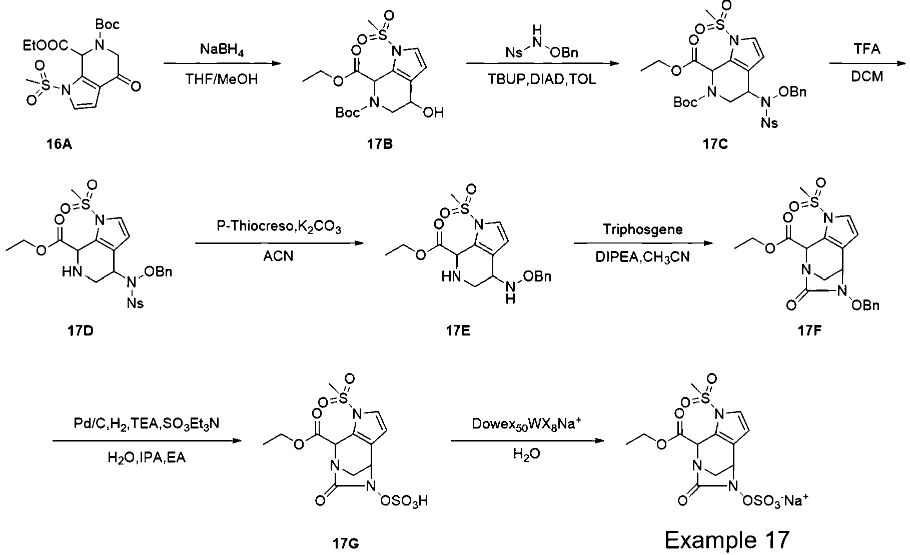

### Synthesis of Intermediate 17B

Intermediate 16A (2 g, 5.18 mmol), tetrahydrofuran (50 mL), and methanol (5 mL) were added to a round-bottom flask. The mixture was cooled to 0-5 °C in an ice bath. Sodium borohydride (0.1 g, 2.59 mmol) was added in two portions. After the addition was complete, the reaction was continued for 0.5 h. TLC monitored the reaction until no starting material remained. Water (50 mL) was added to the reaction solution to quench the reaction. Tetrahydrofuran and methanol were removed by concentration. Ethyl acetate (150 mL) was added, and the aqueous phase was saturated with sodium chloride. After stirring evenly, the mixture was allowed to stand for layer separation. The organic phase was dried over anhydrous sodium sulfate and concentrated to obtain crude intermediate 17B (2 g, yield 99%) as a white oil, which was used directly in the next step.

### Synthesis of Intermediate 17C

Intermediate 17B (2 g, 5.15 mmol), toluene (30 mL), BnONHNs (3.18 g, 10.3 mmol), and tri-n-butylphosphine (2.08 g, 10.3 mmol) were added to a round-bottom flask. The mixture was cooled to 0-5 °C in an ice bath. DIAD (2.08 g, 10.3 mmol) was added dropwise. The mixture was reacted at room temperature overnight. LCMS monitored the reaction until no starting material remained. The reaction solution was concentrated and purified by silica gel chromatography column to obtain intermediate 17C (2.9 g, yield 83%) as a yellow oil.

¹H NMR (400MHz, DMSO-d6) δ 0.82-0.85 (m, 3H), 1.14-1.21 (m, 9H), 3.58 (s, 3H), 4.12-4.17 (m, 2H), 4.18-4.95 (m, 2H), 4.95 (s, 1H), 6.06-6.24 (m, 1H), 6.80-6.88 (m, 2H), 7.18-7.38 (m, 5H), 7.70-7.98 (m, 4H).

### Synthesis of Intermediate 17D

Intermediate 17C (2.9 g, 4.27 mmol) and a solution of TFA in dichloromethane (10 mL TFA dissolved in 40 mL dichloromethane) were added to a round-bottom flask. The mixture was reacted at room temperature for 3 hours. TLC monitored the reaction until no starting material remained. The reaction solution was concentrated, and dichloromethane (100 mL) was used to co-evaporate repeatedly 5 times. Saturated sodium bicarbonate solution (50 mL) and ethyl acetate (100 mL) were added. After stirring evenly, the mixture was allowed to stand for layer separation. The organic phase was washed with saturated brine (50 mL), dried over anhydrous sodium sulfate, and concentrated to obtain crude intermediate 17D (2.67 g, yield 108%) as a yellow oil, which was used directly in the next step.

LCMS: C₂₄H₂₇N₄O₉S₂, Calculated [M+H]⁺ = 579.1, found [M+H]⁺= 579.1.

### Synthesis of Intermediate 17E

Intermediate 17D (2.67 g, 4.61 mmol), acetonitrile (100 mL), p-toluenethiol (1.72 g, 13.83 mmol), and potassium carbonate (1.91 g, 13.83 mmol) were added to a round-bottom flask. The mixture was reacted at room temperature for 6 hours. LCMS monitored the reaction until no starting material remained. The reaction solution was concentrated and purified by silica gel chromatography column to obtain intermediate 17E (1 g, yield 55%) as a yellow oil.

¹H NMR (400MHz, DMSO-d6) δ 1.18-1.23 (m, 3H), 2.64-2.67 (m, 1H), 3.03-3.13 (m, 2H), 3.73-3.75 (m, 1H), 4.02-4.10 (m, 2H), 4.67-4.69 (m, 3H), 6.37 (d, J = 4.0 Hz, 1H), 6.49 (d, J = 8.0 Hz, 1H), 7.13 (d, J = 4.0 Hz, 1H), 7.31-7.37 (m, 5H).

LCMS: C₁₈H₂₃N₄O₄S, Calculated for [M+H]⁺ = 394.0, found [M+H]⁺ = 394.0.

### Synthesis of Intermediate 17F

Intermediate 17E (1 g, 2.54 mmol), acetonitrile (70 mL), and N,N-diisopropylethylamine (1.31 g, 10.16 mmol) were added to a round-bottom flask. The mixture was cooled to 0-5 °C in an ice bath. A solution of triphosgene in acetonitrile (0.3 g triphosgene dissolved in 30 mL acetonitrile) was added dropwise. After the addition was complete, the mixture was reacted at room temperature overnight. LCMS monitored the reaction until no starting material remained. The reaction solution was concentrated and purified by silica gel chromatography column to obtain intermediate 17F (0.75 g, yield 70%) as a white solid.

¹H NMR (400MHz, DMSO-d6) δ 1.25-1.28 (m, 3H), 3.25-3.30 (m, 2H), 3.42 (s, 3H), 4.18-4.31 (m, 2H), 4.54 (s, 1H), 4.93-4.94 (m, 2H), 5.07 (s, 1H), 6.49 (d, J = 4.0 Hz, 1H), 7.24 (d, J = 4.0 Hz, 1H), 7.39-7.46 (m, 5H).

LCMS: C₁₉H₂₁N₄O₅S, Calculated for [M+H]⁺ = 420.1, found [M+H]⁺ = 420.1.

### Synthesis of Intermediate 17G

Intermediate 17F (300 mg, 0.72 mmol), isopropanol (4 mL), ethyl acetate (2 mL), and water (2 mL) were added to a round-bottom flask. Triethylamine (220 mg, 2.22 mmol), triethylamine sulfur trioxide complex (400 mg, 2.22 mmol), and palladium on carbon (60 mg, 0.56 mmol) were added sequentially. The atmosphere was replaced three times with hydrogen protection. The mixture was reacted at room temperature overnight. LCMS monitored the reaction until no starting material remained. The reaction solution was filtered with suction through diatomaceous earth. The filter cake was washed with ethyl acetate (20 mL). The filtrate was concentrated to dryness below 30°C to obtain a crude product. The crude product was purified by reverse phase column chromatography to obtain a solution of the target product in water and acetonitrile. Acetonitrile was removed by concentration below 30°C, and the aqueous solution of intermediate 17G was retained for direct use in the next step.

### Synthesis of Example 17

Dowex 50WX8 resin (10 g) was weighed, added to 2M aqueous sodium hydroxide solution (200 mL), stirred at room temperature for 30 minutes, and filtered. The filter cake was washed with deionized water until the pH was neutral. The filter cake was transferred to the aqueous solution of intermediate 17G and stirred at room temperature for 4 hours. The reaction solution was then filtered, and the filtrate was lyophilized to obtain Example 17 (30 mg, total yield over two steps 10.25%) as a light yellow solid.

¹H NMR (400MHz, DMSO-d6) δ 1.26-1.29 (m, 3H), 3.28-3.35 (m, 2H), 3.42 (s, 3H), 4.19-4.32 (m, 2H), 4.72 (d, J = 4.0 Hz, 1H), 5.07 (s, 1H), 6.45 (d, J = 4.0 Hz, 1H), 7.25 (d, J = 4.0 Hz, 1H). HRMS (ESI)⁻: calcd for C₁₂H₁₃N₃O₉S₂, [M-Na]⁻: 408.0177, found: 408.0158.

### Example 18

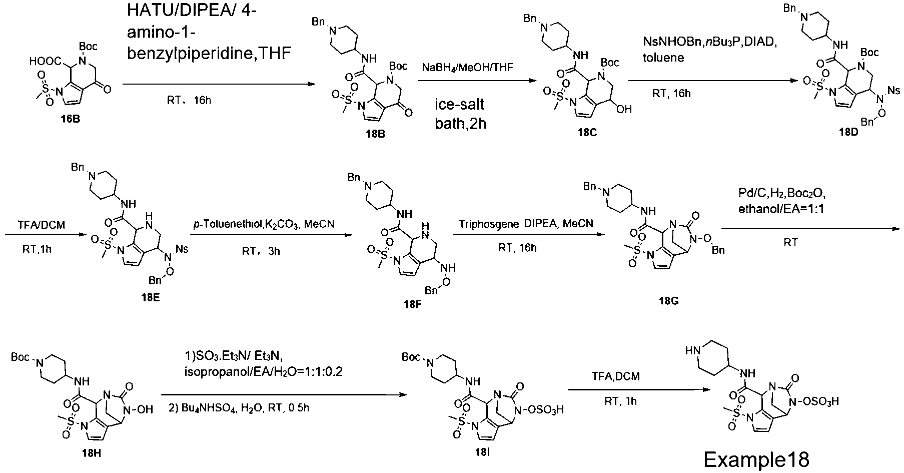

### Synthesis of Intermediate 18B:

Intermediate 16B (10 g, 27.9 mmol), THF (100 mL), HATU (13.79 g, 36.27 mmol), and N,N-diisopropylethylamine (7.21 g, 55.8 mmol) were added to a 250 mL eggplant-shaped flask. The mixture was stirred at room temperature for 0.5 h. 4-Amino-1-benzylpiperidine (5.84 g, 30.69 mmol) was added, and the mixture was stirred at room temperature overnight. LCMS monitoring showed no starting material remained. 100 mL of water and 200 mL of ethyl acetate were added to the reaction solution. After stirring evenly, the mixture was allowed to stand for layer separation. The organic phase was washed successively with 100 mL of 1M aqueous hydrochloric acid solution, 100 mL of saturated aqueous sodium bicarbonate solution, and 100 mL of saturated brine, dried over anhydrous sodium sulfate, and the solvent was evaporated under reduced pressure. Purification by silica gel chromatography column gave 4.4 g of the target product as a gray solid, yield 29.7%.

LCMS: Calculated for C₂₆H₃₄N₄O₆S [M] = 530.22; found [M+H]⁺ = 531.5.

### Synthesis of Intermediate 18C:

Intermediate 18B (4.4 g, 8.29 mmol), THF (45 mL), and methanol (5 mL) were added to a 250 mL eggplant-shaped flask. The mixture was cooled to 0-5°C in an ice bath. Sodium borohydride (0.38 g, 9.95 mmol) was added in two portions. After the addition was complete, the reaction was continued for 2 h. TLC monitored the reaction until no starting material remained. 50 mL of water was added to the reaction solution to quench the reaction. THF and methanol were removed by concentration. 150 mL of ethyl acetate was added, and the aqueous phase was saturated with sodium chloride. After stirring evenly, the mixture was allowed to stand for layer separation. The organic phase was dried over anhydrous sodium sulfate and concentrated to obtain 4.2 g of crude target product as a yellow solid, yield 95%.

¹H NMR (400MHz, DMSO-d6) δ: 1.43 (s, 9H), 1.59-1.72 (m, 6H), 2.13-2.20 (m, 2H), 2.75-2.82 (m, 3H), 3.47-3.69 (m, 5H), 4.38-4.53 (m, 1H), 5.53-5.80 (m, 1H), 6.40-6.67 (m, 3H), 7.29-7.60 (m, 6H), 8.14-8.25 (m, 1H).

### Synthesis of Intermediate 18D:

Intermediate 18C (4.2 g, 7.89 mmol), toluene (45 mL), BnONHNs (4.87 g, 15.78 mmol), and tri-n-butylphosphine (3.19 g, 15.78 mmol) were added to a 100 mL eggplant-shaped flask. The mixture was cooled to 0-5°C in an ice bath. DIAD (3.19 g, 15.78 mmol) was added dropwise. The mixture was reacted at room temperature overnight. LCMS monitored the reaction until no starting material remained. The reaction solution was concentrated and purified by silica gel chromatography column to obtain 3.1 g of the target product as a yellow oil, yield 47%.

LCMS: Calculated for C₃₉H₄₆N₆O₁₀S₂ , [M] = 822.27, found [M+H]⁺ = 823.3.

### Synthesis of Intermediate 18E:

Intermediate 18D (3.1 g, 3.77 mmol) and a solution of TFA in DCM (8 mL TFA dissolved in 40 mL DCM) were added to a 100 mL eggplant-shaped flask. The mixture was reacted at room temperature for 3 h. TLC monitored the reaction until no starting material remained. The reaction solution was concentrated, and DCM (100 mL) was used to co-evaporate repeatedly 5 times. 50 mL of saturated sodium bicarbonate and 100 mL of ethyl acetate were added. After stirring evenly, the mixture was allowed to stand for layer separation. The organic phase was washed with 50 mL of saturated brine, dried over anhydrous sodium sulfate, and concentrated to obtain a total of 2.1 g of crude target product as a yellow oil, yield 77%.

LCMS: Calculated for C₃₄H₃₈N₆O₈S₂ [M] = 722.22; found [M+H]⁺ = 723.0.

### Synthesis of Intermediate 18F:

Intermediate 18E (2.1 g, 2.91 mmol), 10 mL of acetonitrile, 10 mL of methanol, p-toluenethiol (1.08 g, 8.73 mmol), and potassium carbonate (1.21 g, 8.73 mmol) were added to a 100 mL eggplant-shaped flask. The mixture was reacted at room temperature for 3 h. LCMS monitored the reaction until no starting material remained. The reaction solution was concentrated and purified by silica gel chromatography column to obtain 1.1 g of the target product as a yellow oil, yield 70%.

LCMS: Calculated for C₂₈H₃₅N₅O₄S, [M] = 537.24; found [M+H]⁺ = 538.0.

### Synthesis of Intermediate 18G:

Intermediate 18F (1.1 g, 2.05 mmol), acetonitrile (70 mL), and N,N-diisopropylethylamine (1.06 g, 8.2 mmol) were added to a 250 mL eggplant-shaped flask. The mixture was cooled to 0-5°C in an ice bath. A solution of triphosgene in acetonitrile (0.24 g triphosgene dissolved in 30 mL acetonitrile, added at a rate of one drop every 2-3 seconds) was added dropwise. After the addition was complete, the mixture was reacted at room temperature overnight. LCMS monitored the reaction until no starting material remained. The reaction solution was concentrated and purified by preparative TLC to obtain 0.61 g of the target product as a light yellow solid, yield 53%.

¹H NMR (400MHz, DMSO-d6) δ 1.48-1.55 (m, 2H), 1.68-1.74 (m, 2H), 2.01-2.03 (m, 2H), 2.77-2.80 (m, 2H), 3.20-3.38 (m, 1H), 3.51-3.57 (m, 4H), 4.48 (s, 1H), 4.88-4.95 (m, 2H), 5.08 (s, 1H), 6.41 (d, J = 4.0 Hz, 1H), 7.12 (d, J = 4.0 Hz, 1H), 7.26-7.45 (m, 10H), 8.55 (d, J = 4.0 Hz, 1H).

LCMS: Calculated for C₂₉H₃₃N₅O₅S, [M] = 563.22; found [M+H]⁺ = 564.3.

### Synthesis of Intermediate 18H:

Intermediate 18G (0.5 g, 0.89 mmol), ethanol (10 mL), ethyl acetate (10 mL), di-tert-butyl dicarbonate (0.29 g, 1.33 mmol), and palladium on carbon (167 mg, 1.58 mmol) were added to a 100 mL eggplant-shaped flask. The atmosphere was replaced five times with hydrogen protection. The mixture was reacted at room temperature overnight. LCMS monitored the reaction until no starting material remained. The reaction solution was filtered through diatomaceous earth. The filter cake was washed with 20 mL of ethyl acetate and 20 mL of ethanol. The filtrate was concentrated below 30°C and purified by preparative TLC to obtain a total of 45 mg of the target product as a yellow oil, yield 10%.

LCMS: Calculated for C₂₀H₂₉N₅O₇S, [M] = 483.18; found [M+H]⁺ = 484.1.

### Synthesis of Intermediate 18I:

Intermediate 18H (45 mg, 0.093 mmol), triethylamine sulfur trioxide complex (40 mg, 0.22 mmol), isopropanol (1 mL), ethyl acetate (1 mL), water (0.2 mL), triethylamine (28 mg, 0.28 mmol), and palladium on carbon (51 mg, 0.28 mmol) were added to a 25 mL eggplant-shaped flask. The atmosphere was replaced three times with nitrogen protection. The mixture was reacted at room temperature overnight. LCMS monitored the reaction until no starting material remained. 1. The reaction solution was concentrated below 30°C to obtain crude product 1. 10 mL of water and 10 mL of ethyl acetate were added to crude product 1. After stirring evenly, the mixture was allowed to stand for layer separation. The aqueous phase was extracted once more with 10 mL of ethyl acetate. Tetrabutylammonium hydrogen sulfate (315 mg, 0.93 mmol) was added to the aqueous phase, and the mixture was stirred at room temperature for 0.5 h. The mixture was extracted with DCM (30 mL x 2). The organic phase was concentrated to dryness to obtain crude product 2. Crude product 2 was purified by HPLC preparative separation and lyophilized to obtain 25 mg of the target product as a white solid, yield 33%.

¹H NMR (400MHz, DMSO-d6) δ 0.93-0.97 (m, 14H), 1.28-1.37 (m, 11H), 1.55 (m, 12H), 1.58-1.63 (m, 9H), 1.70-1.75 (m, 2H), 2.87 (br, 2H), 3.16-3.20 (m, 10H), 3.41-3.44 (m, 4H), 3.77-3.91 (m, 3H), 4.65 (d, J = 4.0 Hz, 1H), 5.07 (s, 1H), 6.36 (d, J = 4.0 Hz, 1H), 7.13 (d, J = 4.0 Hz, 1H), 8.63 (d, J = 8.0 Hz, 1H).

LCMS: Calculated for C₂₀H₂₉N₅O₁₀S₂ , [M] = 563.14; found [M-H]⁻ = 562.1.

### Synthesis of Example 18:

Intermediate 18I (23 mg, 0.041 mmol) and a solution of TFA in DCM (1 mL TFA dissolved in 5 mL DCM) were added to a 25 mL eggplant-shaped flask. The mixture was reacted at room temperature for 1 h. LCMS monitored the reaction until no starting material remained. The reaction solution was concentrated, and 100 mL of DCM was used to co-evaporate repeatedly 5 times. The residue was concentrated, purified by HPLC preparative column separation, and lyophilized to obtain a total of 3.5 mg of the target product as a white solid, yield 18%.

¹H NMR (400MHz, DMSO-d6) δ 1.67-1.69 (m, 2H), 1.91-2.00 (m, 3H), 2.50 (m, 1H), 2.68-2.70 (m, 1H), 3.87-3.91 (m, 2H), 4.65 (d, J = 4.0 Hz, 1H), 5.09 (s, 1H), 6.38 (d, J = 4.0 Hz, 1H), 7.14 (d, J = 4.0 Hz, 1H), 8.23 (br, 1H), 8.82 (d, J = 8.0 Hz, 1H). LCMS: Calculated for C₁₅H₂₁N₅O₈S₂ [M] = 463.08; found [M-H]⁻ = 462.1.

### Example 19

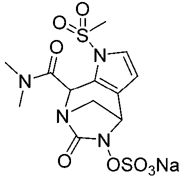

The synthetic method for Example 19 was similar to that of Example 18. ¹H NMR (400 MHz, DMSO) δ 7.14 (d, J = 6.0 Hz, 1H), 6.39 (d, J = 3.3 Hz, 1H), 5.36 (s, 1H), 4.67 (d, J = 1.6 Hz, 1H), 3.40 (s, 3H), 3.20 (s, 3H), 2.94 (s, 3H). LCMS: Calculated for C₁₂H₁₅N₄O₈S₂ , [M-Na]⁻ = 407.03; found 407.1.

### General Synthetic Route for Examples 20-23

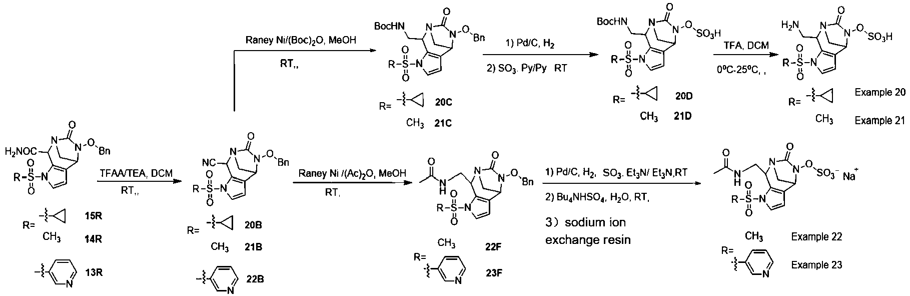

### Example 20

### Synthesis of Intermediate 20B:

Starting material intermediate 15R (1.40 g, 3.36 mmol) was dissolved in 14 mL of dichloromethane. Triethylamine (33.6 mmol, 3.4 g) was added. The reaction was cooled to 0-5 °C, and trifluoroacetic anhydride (6.72 mmol, 1.41 g) was added dropwise. After the addition was complete, the reaction was warmed to room temperature and stirred at that temperature for 2 hours. The reaction was monitored by LCMS. After the reaction was complete, the reaction solution was poured into ice water (30 mL) to quench. The layers were separated. The aqueous phase was extracted with dichloromethane (10 mL x 2). The organic phases were combined, washed with saturated brine (20 mL x 3), dried over anhydrous sodium sulfate, filtered, and concentrated to obtain a crude product. The crude product was purified by prep-TLC to obtain intermediate 20B as a white foam solid (860 mg, yield 64.2%).

¹H NMR (400 MHz, DMSO-d6) δ 7.48 - 7.35 (m, 5H), 7.36 (d, J = 3.3 Hz, 1H), 6.54 (d, J = 3.3 Hz, 1H), 5.63 (s, 1H), 5.00 - 4.88 (m, 2H), 4.61 (d, J = 2.1 Hz, 1H), 3.61 - 3.49 (m, 2H), 3.17 - 3.04 (m, 1H), 1.36 - 1.15 (m, 4H).

LCMS: Calculated for C₁₉H₁₈N₄O₄S, M = 398.10; found [M+H]⁺ = 399.0.

### Synthesis of Intermediate 20C:

Starting material intermediate 20B (400 mg, 1.0 mmol) was dissolved in methanol (8 mL). Di-tert-butyl dicarbonate (436 mg, 2.0 mmol) was added, followed by Raney nickel (200 mg). The atmosphere was replaced with hydrogen three times, and the reaction was stirred at room temperature for 6 hours. The reaction was monitored by LCMS. After the reaction was complete, the mixture was filtered through diatomaceous earth. The filter cake was washed with methanol (10 mL x 3) and discarded (quenched with dilute hydrochloric acid). The filtrate was concentrated to remove the solvent, obtaining a crude product. The crude product was purified by prep-TLC to obtain intermediate 20C as a yellow solid (200 mg, 39.6%).

¹H NMR (400 MHz, DMSO-d6) δ 7.49 - 7.34 (m, 5H), 7.14 (d, J = 3.2 Hz, 1H), 6.96 (t, J = 5.7 Hz, 1H), 6.42 (d, J = 3.3 Hz, 1H), 4.91 (s, 2H), 4.59 - 4.50 (m, 1H), 4.41 (d, J = 1.9 Hz, 1H), 3.81 - 3.7 (m, 1H), 3.53 - 3.38 (m, 2H), 3.17 - 2.88 (m, 2H), 1.41 (s, 9H), 1.26 - 1.04 (m, 4H).

LCMS: Calculated for C₂₄H₃₀N₄O₆S, M = 502.19; found [M+H]⁺ = 503.1.

### Synthesis of Intermediate 20D:

In a 10 mL three-necked flask, compound 20C (250 mg, 0.50 mmol) was dissolved in 3 mL of methanol. Palladium catalyst (100 mg) was added, and the mixture was stirred at room temperature under hydrogen protection. After 2 h, LCMS monitoring showed the reaction was complete. The reaction solution was filtered under reduced pressure. The filtrate was directly concentrated to obtain a crude yellow oil. Purification by column chromatography yielded 180 mg of a white solid, yield 89.3%. LCMS: Calculated for C₁₇H₂₄N₄O₆S, M = 412.14; found [M+1]⁺ = 413.0.

The above white solid (180 mg, 0.44 mmol) and sulfur trioxide pyridine complex (208 mg, 1.3 mmol) were added to a 25 mL three-necked round-bottom flask. 3 mL of ultra-dry pyridine was added. The mixture was stirred at room temperature under nitrogen protection, forming a yellow suspension.

After overnight reaction, LCMS monitoring showed the reaction was complete. The mixture was concentrated directly at room temperature to remove pyridine. Dichloromethane was added to the reaction solution to dissolve the product. The mixture was filtered under reduced pressure to remove solids. The filtrate was collected and concentrated to obtain crude product 20D as a brown oil (300 mg). The crude product was used directly in the next step without further purification.

LCMS: Calculated for C₁₇H₂₄N₄O₉S₂, M = 492.10, found [M-H]⁻ = 491.0.

### Synthesis of Example 20:

Crude compound 20D (300 mg) was added to a 25 mL three-necked flask and dissolved in 3 mL of dichloromethane. The mixture was cooled to 0°C in an ice bath. Trifluoroacetic acid (2 mL) was slowly added dropwise. After the addition was complete, the mixture was naturally warmed to room temperature and stirred. The solution became a brown clear solution.

After 30 min, LCMS monitoring showed the reaction was complete. The mixture was concentrated at low temperature to obtain a crude brown oil. 5 mL of acetonitrile was added to the concentrated solution, and the mixture was stirred. A solid precipitated. The mixture was filtered under reduced pressure, and the filter cake was collected. The filter cake was washed with dichloromethane, methanol, and acetonitrile to obtain Example 20 as a white powder solid (90 mg). HPLC analysis showed a purity of 96.8%.

¹H NMR (400 MHz, DMSO-d6) δ 8.17 (s, 3H), 7.23 (d, J = 3.3 Hz, 1H), 6.46 (d, J = 3.3 Hz, 1H), 4.77 (dd, J = 11.2, 3.7 Hz, 1H), 4.70 (d, J = 2.5 Hz, 1H), 3.57 (d, J = 11.6 Hz, 1H), 3.54 - 3.47 (m, 1H), 3.35 - 3.28 (m, 2H), 3.00 - 2.90 (m, 1H), 1.30 - 1.02 (m, 4H). LCMS: Calculated for C₁₂H₁₆N₄O₇S₂, M = 392.05, found [M-H]⁻ = 391.0.

### Example 21

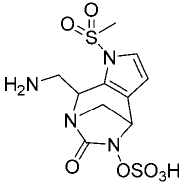

The synthetic method for Example 21 was similar to that of Example 20.

¹H NMR (400 MHz, DMSO) δ 7.76 (s, 3H), 7.22 (d, J = 3.2 Hz, 1H), 6.45 (d, J = 3.3 Hz, 1H), 4.74 (dd, J = 11.0, 3.8 Hz, 1H), 4.69 (d, J = 2.5 Hz, 1H), 3.66 - 3.51 (m, 2H), 3.44 (s, 4H), 3.30 (d, J = 2.6 Hz, 2H). LCMS: M=C₁₀H₁₄N₄O₇S, Calculated for [M-H]⁻ = 365.03; found [M-H]⁻ = 365.1.

### Example 22

### Synthesis of Intermediate 21B:

In a 100 mL three-necked flask, compound 14R (3.7 g, 9.48 mmol) was added and dissolved in 40 mL of dichloromethane. Triethylamine (9.8 g, 94.80 mmol) was added to the reaction solution, resulting in a yellow clear solution. The mixture was cooled to 0°C and stirred for 5 minutes. Trifluoroacetic anhydride (4.0 g, 18.9 mmol) was added dropwise to the reaction solution. After the addition was complete, the mixture was naturally warmed to room temperature and stirred. The solution became a yellow clear solution. After 1 h of reaction, LCMS analysis showed the reaction was complete. 40 mL of water was added to the reaction solution, and the mixture was stirred for 10 minutes. The layers were separated, and the dichloromethane phase was collected. The aqueous phase was extracted again with 30 mL of dichloromethane. The dichloromethane layers were combined, washed with water and saturated brine, dried over anhydrous Na2S04, and concentrated to obtain a crude yellow oil. Purification by silica gel chromatography column gave compound 21B as a brown foam solid (3.0 g, yield 85.2%).

¹H NMR (400 MHz, DMSO-d6) δ 7.48 - 7.33 (m, 5H), 7.31 (d, J = 4.0 Hz, 1H), 6.54 (d, J = 3.3 Hz, 1H), 5.66 (s, 1H), 4.97 - 4.88 (m, 2H), 4.61 (d, J = 2.1 Hz, 1H), 3.60 - 3.47 (m, 5H).

LCMS: M=C₁₇H₁₆N₄O₄S, Calculated for [M+H]⁺ = 373.09; found [M+H]⁺ = 373.1.

### Synthesis of Intermediate 22F

At room temperature, intermediate 21B (200 mg, 0.54 mmol) was added to methanol (4 mL) and stirred to dissolve. Acetic anhydride (110 mg, 1.18 mmol) and Raney nickel (95 mg, 1.62 mmol) were added successively. The atmosphere was replaced with hydrogen three times, and the reaction was stirred at room temperature overnight. LCMS monitoring showed the starting material had disappeared. The reaction solution was filtered with suction. The filtrate was concentrated and purified by column chromatography to obtain 102 mg of a white product (45.1 %).

LCMS: Calculated for C₁₉H₂₀N₄O₅S, [M] = 418.13, found: [M+H]⁺ = 419.1.

### Synthesis of Example 22

At room temperature, intermediate 22F (0.1 g, 0.24 mmol) was dissolved in isopropanol (1.5 mL) and ethyl acetate (1.5 mL). Palladium on carbon (0.097 g, 0.91 mmol), triethylamine sulfur trioxide complex (0.087 g, 0.48 mmol), and triethylamine (0.061 g, 0.60 mmol) were added and stirred to dissolve. The atmosphere was replaced with hydrogen three times. After 3 hours of reaction, LCMS monitoring showed the starting material had disappeared. The reaction solution was filtered with suction at room temperature, washed with deionized water (5 mL). The filtrate was concentrated at low temperature to obtain a crude product. The crude product was dissolved in deionized water (5 mL) and extracted with ethyl acetate (5 mL) to remove impurities. 10 eq of solid tetrabutylammonium hydrogen sulfate was added and stirred for 1 hour. The mixture was extracted with dry dichloromethane (5 mL), concentrated at low temperature, and purified by preparative separation to obtain 10 mg of product after lyophilization. 2 g of Dowex cation exchange resin was taken, activated with sodium hydroxide (20 mL, 2M) for 1 hour, and filtered. The filter cake was the exchanged resin, which was washed with deionized water until the filtrate pH was neutral. The lyophilized product and the ion exchange resin were dissolved together in 5 mL of deionized water, stirred at room temperature for 5 hours, then rinsed 5 times on a silica gel column. The resulting mobile phase was lyophilized to obtain the target compound, Example 22 (6 mg, 5.8%).

¹H NMR (400 MHz, DMSO-d6) δ = 8.02 (s, 1H), 7.07 (d, J = 0 Hz, 1H), 6.29 (d, J = 4 Hz, 1H), 4.53 (d, J = 4 Hz, 1H), 4.42 (t, J = 16 Hz, 1H), 3.42 (d, J = 12 Hz, 2H), 3.35 (s, 3H), 3.12 (d, J = 8 Hz, 2H), 1.79 (s, 3H) ppm.

LCMS: Calculated for C₁₂H₁₅N₄NaO₈S₂, [M-H]⁻ = 407.03, found: 407.1.

### Example 23

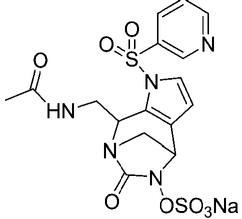

The synthetic method for Example 23 was the same as for Example 22. ¹H-NMR (DMSO-d6, 400MHz): δ 1.83-1.90 (m, 3H), 3.17-3.21 (m, 1H), 3.44-3.51 (m, 2H), 3.80-3.86 (m, 1H), 4.51-4.55 (m, 1H), 4.57-4.59 (m, 1H), 6.43 (d, J = 4.0 Hz, 1H), 7.43 (d, J = 4.0 Hz, 1H), 7.70-7.73 (m, 1H), 8.12-8.15 (m, 1H), 8.27-8.30 (m, 1H), 8.95 (d, J = 4.0 Hz, 1H), 9.11 (d, J = 4.0 Hz, 1H).

LCMS: C₁₆H₁₆N₅O₈S₂⁻, Calculated [M-Na]⁻ = 470.04, found [M-Na]⁻ = 470.0.

### General Synthetic Route for Examples 24-26

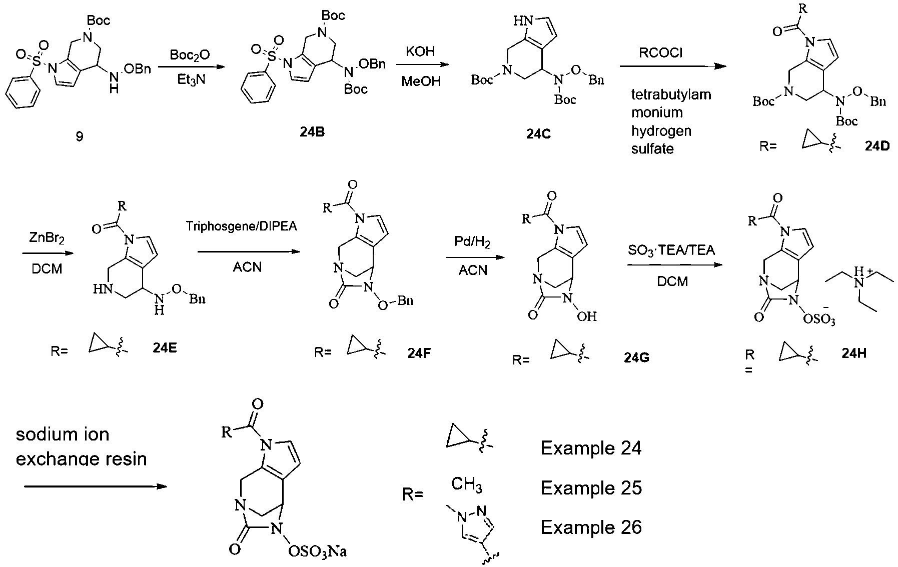

### Example 24

### Synthesis of Intermediate 24B

To a solution of intermediate 9 (52 g, 107.53 mmol) in triethylamine (500 ml) was added di-tert-butyl dicarbonate (117.34 g, 537.65 mmol). The mixture was heated to 100°C and reacted under reflux for 7 h. TLC monitoring showed the starting material had reacted completely. The system was concentrated, and silica gel column chromatography gave intermediate 24B as a yellow oil (30 g, 47.8%).

LC-MS: Calculated for C₃₀H₃₇N₃O₇S, M = 583.24, found [M+H]⁺ = 584.4.

### Synthesis of Intermediate 24C

Intermediate 24B (28 g, 47.97 mmol) was dissolved in a mixed solution of methanol and water (methanol/water = 10:1, methanol: 300 mL, water: 30 mL). Potassium hydroxide (13.4 g, 240 mmol) was added to the mixed solution. Under nitrogen protection, the mixture was reacted at 70°C for 3 h. LC-MS showed the starting material had reacted completely. Dilute hydrochloric acid was added to adjust the system to neutral. The mixture was concentrated under reduced pressure to remove methanol. Ethyl acetate (150 mL) was added for extraction. After separating the organic phase, the aqueous phase was extracted again with ethyl acetate (50 mL × 2). The organic phases were combined, washed with brine, dried over anhydrous sodium sulfate, and concentrated. Silica gel column chromatography gave intermediate 24C as a yellow oil (16.4 g, 77.1%).

LC-MS: Calculated for C₂₄H₃₃N₃O₅, M = 443.24, found [M+H]⁺ = 444.3.

### Synthesis of Intermediate 24D

At 0 °C, to a solution of intermediate 24C (1 g, 2.25 mmol), sodium hydroxide (0.09 g, 2.25 mmol), and tetrabutylammonium hydrogen sulfate (0.038 g, 0.11 mmol) in dichloromethane (30 mL) was slowly added dropwise cyclopropanecarbonyl chloride (0.35 g, 0.31 mL, 23.38 mmol). The reaction solution was warmed to room temperature and stirred overnight. TLC monitoring showed the starting material had reacted completely. The mixture was extracted with water and then separated. The organic phase was concentrated. Silica gel column chromatography gave intermediate 24D as a yellow oil (0.42 g, 36.41 %).

LC-MS: Calculated for C₂₃H₃₇N₃O₆, M = 511.27, found [M+H]⁺ = 512.3.

¹H NMR (400 MHz, DMSO-d6): δ 7.72 (d, J = 3.5 Hz, 1H), 7.29 (s, 3H), 7.17-7.08 (m, 2H), 6.32-6.28 (m, 1H), 4.96 (s, 1H), 4.85 - 4.41 (m, 4H), 3.92 - 3.77 (m, 1H), 3.71 - 3.45 (m, 1H), 2.66 - 2.57 (m, 1H), 1.51 (s, 9H), 1.35 (s, 9H), 1.15 - 1.05 (m, 4H).

### Synthesis of Intermediate 24E

To a solution of intermediate 24D (0.6 g, 1.17 mmol) in DCM (30 mL) was added zinc bromide (2.64 g, 11.7 mmol). After reacting at room temperature for 3 hours, TLC monitoring showed the starting material had reacted completely. Sodium bicarbonate suspension was added to the reaction solution, stirred for 1 hour, and then filtered. The filtrate was dried over anhydrous sodium sulfate and concentrated under reduced pressure to give intermediate 24E as a yellow oil (0.37 g, 100%), which was used directly in the next step.

LC-MS: Calculated for C₁₈H₂₁N₃O₂, M = 311.16, found [M+H]⁺ = 312.1.

¹H NMR (400 MHz, DMSO-d6): δ 7.55 (d, J = 3.4 Hz, 1H), 7.40 - 7.26 (m, 5H), 6.58 (d, J = 8.0 Hz, 1H), 6.29 (d, J = 3.3 Hz, 1H), 4.66 (s, 2H), 3.94 (d, J = 17.7 Hz, 1H), 3.82 - 3.73 (m, 2H), 3.01 (dd, J = 13.3, 3.1 Hz, 1H), 2.67 (dd, J = 13.3, 3.6 Hz, 1H), 2.58 - 2.52 (m, 1H), 1.06 - 0.98 (m, 4H).

### Synthesis of Intermediate 24F

At room temperature, triphosgene (0.14 g, 0.48 mmol) was dissolved in acetonitrile (5 mL). At -20 °C, this solution was added dropwise to a solution of intermediate 24E (0.37 g, 1.19 mmol) and DIPEA (0.62 g, 0.79 mL, 4.76 mmol) in acetonitrile (10 mL). After reacting at room temperature for 2 hours, LC-MS showed the starting material had reacted completely. Ethyl acetate (10 mL) and water (10 mL) were added for extraction. The organic phase was separated, and the aqueous phase was extracted twice more with ethyl acetate (10 mL × 2). The organic phases were combined, washed with brine, dried over sodium sulfate, filtered, and concentrated. Silica gel column chromatography gave intermediate 24F as a yellow oil (0.26 g, 63%).

LC-MS: Calculated for C₁₉H₁₉N₃O₃, M = 337.14, found [M+H]⁺ = 338.1.

¹H NMR (400 MHz, DMSO-d6): δ 7.61 (d, J = 3.4 Hz, 1H), 7.41 (m, 7.46-7.35 Hz, 5H), 6.36 (d, J = 3.4 Hz, 1H), 4.91 (s, 2H), 4.46 - 4.32 (m, 3H), 3.40 - 3.35 (m, 1H), 3.22 (d, J = 10.9 Hz, 1H), 2.63 - 2.54 (m, 1H), 1.15 - 0.97 (m, 4H).

### Synthesis of Intermediate 24G

To a solution of intermediate 24F (260 mg, 0.76 mmol) in acetonitrile (20 mL) was added palladium on carbon (10 wt%, 260 mg). The atmosphere was replaced twice with a double-layer hydrogen balloon. After stirring at ambient temperature for 5 hours, LCMS monitoring showed the starting material had reacted completely. The mixture was filtered, and the filtrate was concentrated under reduced pressure to give intermediate 24G as a white solid (0.15 g, 80%), which was used directly in the next step.

LC-MS: Calculated for C₁₂H₁₃N₃O₃, M = 247.1, found [M+H]⁺ = 248.1.

¹H NMR (400 MHz, DMSO-d6) δ 9.44 (s, 1H), 7.59 (d, J = 3.4 Hz, 1H), 6.36 (d, J = 3.4 Hz, 1H), 4.37 (s, 2H), 4.28 (d, J = 2.7 Hz, 1H), 3.45-3.42 (m, 1H), 3.17 (d, J = 10.7 Hz, 1H), 2.61-2.55 (m, 1H), 1.10-1.02 (m, J = 8.0, 4.8, 4.1 Hz, 4H).

### Synthesis of Intermediate 24H

At room temperature, to a solution of intermediate 24G (0.15 g, 0.61 mmol) in DCM (10 mL) was added triethylamine (0.19 g, 0.25 mL, 1.83 mmol) and triethylamine sulfur trioxide complex (0.33 g, 1.83 mmol). After reacting at room temperature for 3 hours, LCMS monitoring showed the starting material had reacted completely. The reaction solution was concentrated under reduced pressure, and silica gel column chromatography (DCM/MeOH = 0-10%) gave intermediate 24H as a white solid.

LC-MS: Calculated for C₁₂H₁₂N₃O₆S, [M-H]⁻ = 326.04, found [M-H]⁻ = 326.0.

### Synthesis of Example 24

Intermediate 24H was dissolved in a mixed solvent of acetonitrile (10 mL) and water (5 mL). Sodium ion exchange resin was added, and the mixture was stirred at ambient temperature for 3 hours, then filtered. The filter cake was washed with water and acetone. The filtrate was lyophilized, purified by prep-HPLC, and lyophilized again to obtain Example 24 as a white solid (135 mg, 64%).

¹H NMR (400 MHz, DMSO-d6) δ 7.62 (d, J = 3.4 Hz, 1H), 6.33 (d, J = 3.3 Hz, 1H), 4.62 (d, J = 2.6 Hz, 1H), 4.45 - 4.32 (m, 2H), 3.47 (dd, J = 10.9, 2.9 Hz, 1H), 3.22 (d, J = 10.8 Hz, 1H), 2.63-2.57 (m, 1H), 1.12 - 1.01 (m, 4H). LC-MS: Calculated for C₁₂H₁₂N₃O₆S⁻, [M-Na]⁻ = 326.04, found 326.0.

### Example 25

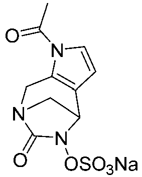

The synthetic method for Example 25 was similar to that of Example 24. LC-MS: Calculated for C₁₀H₁₀N₃O₆S, [M-Na]⁻ = 300.0, found 300.03.

### Example 26

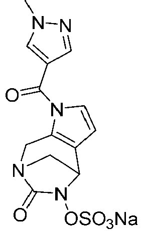

The synthetic method for Example 26 was similar to that of Example 24. LC-MS: Calculated for C₁₃H₁₂N₅O₆S, [M-Na]⁻ = 366.05, found 366.02.

The beneficial effects of the present invention are demonstrated through the following specific test examples.

### Test Example 1: Antibacterial Activity Test

The minimum inhibitory concentration (MIC) of the Example compounds (at a concentration of 4 mg/L) in combination with β-lactam antibiotics (ceftazidime, cefepime, meropenem, imipenem, aztreonam) against bacterial strains was tested using the broth microdilution method recommended by the Clinical and Laboratory Standards Institute (CLSI, formerly NCCLs) [Methods for Dilution Antimicrobial Susceptibility Tests for Bacteria That Grow Aerobically; Approved Standard-tenth Edition M07-A10, 2015]. The specific test materials and procedures are as follows.

### Bacterial Strain Sources:

The standard strains used in this experiment were purchased ATCC strains. The clinical strains used were strains resistant to some or all marketed β-lactam antibiotics, isolated and identified by domestic public hospitals, and the types of β-lactamases they express were determined. Before the experiment, each bacterial strain was purified by streaking single colonies on an agar plate, and fresh bacterial cultures grown overnight at 37°C were appropriately diluted for the experiment.

### Culture Medium and Conditions

CAMHB (Cation Adjusted Mueller-Hinton) medium, incubated at 35-37°C in air for 16-20 h.

CAMHB Formula: Acid-hydrolyzed casein (17.5 g/L), beef extract (3.0 g/L), soluble starch (1.5 g/L), calcium ions (20-25 mg/L), magnesium ions (10-12.5 mg/L).

### Preparation of Test Samples

Stock solutions at 4 times the working concentration were prepared in CAMHB broth for the combinations of antibiotics (ceftazidime, cefepime, meropenem, aztreonam) and Example compounds. The final concentrations of antibiotics in the stock solutions were 256, 128, 64, 32, 16, 8, 4, 2, 1, 0.5, 0.25, 0.125 mg/L, respectively. The final concentration of avibactam, relebactam, or Example compounds was 4 mg/L.

### Preparation of Inoculum

Several colonies were picked from an 18-24 h cultured agar plate and suspended directly in sterile physiological saline to prepare a bacterial suspension. The turbidity of the suspension was adjusted to 0.5 McFarland standard. The adjusted bacterial suspension was diluted with CAMHB broth to (4-8) × 10⁵ CFU/mL and used immediately.

### Sample Addition and Inoculation

Test Group: 50 µL of the antibiotic (ceftazidime, cefepime, meropenem, imipenem, aztreonam) stock solution and 50 µL of the β-lactamase inhibitor (avibactam, relebactam, or Example compound) stock solution were added to wells 1 to 12 of a sterile 96-well polystyrene plate. Then, 100 µL of CAMHB containing the test bacterial suspension was added to each well, making a total volume of 200 µL per well. The final drug concentrations in the wells were 64, 32, 16, 8, 4, 2, 1, 0.5, 0.25, 0.125, 0.06, 0.03 mg/L, respectively. The final concentration of the Example compounds or known β-lactamase inhibitors was 4 mg/L. The final bacterial suspension concentration in all wells was (2-4) × 10⁵ CFU/mL.

Ceftazidime Alone Group: The addition method was the same as above. Each well contained only ceftazidime, with final concentrations of 64, 32, 16, 8, 4, 2, 1, 0.5, 0.25, 0.125, 0.06, 0.03 mg/L, respectively. The final bacterial suspension concentration was (2-4) × 10⁵ CFU/mL, without β-lactamase inhibitor.

Bacterial Control Group: Wells were inoculated only with the bacterial suspension at a final concentration of (2-4) × 10⁵ CFU/mL, without any drug.

### Incubation

The inoculated 96-well polystyrene plates were sealed and placed in an incubator at 35-37°C for 16-20 h before reading the results.

### MIC Endpoint Reading

The MIC (Minimum Inhibitory Concentration) was determined by visual observation as the lowest concentration of the drug that completely inhibited bacterial growth in the well.

**Table 1. Antibacterial Activity (MIC₅₀, mg/L) of Example Compounds in Combination with Ceftazidime**

| ATCC Standard Resistant Strains | E. coli ATCC 25922 | E. coli ATCC 35218 | P. aeruginosa ATCC 27853 | E. Coli NCTC 13353 | K.Pneumoniae BAA-1705 |
|---|---|---|---|---|---|
| β-Lactamase Expressed by Strain | - | TEM-1 | AmpC | CTX-M-15 | KPC-2 |
| Ceftazidime | ++ | ++ | + | - | - |
| Ceftazidime + Avibactam | ++ | ++ | ++ | ++ | ++ |
| Ceftazidime + Example 1 Compound | ++ | ++ | + | - | - |
| Ceftazidime + Example 2 Compound | +++ | +++ | ++ | +++ | ++ |
| Ceftazidime + Example 3 Compound | +++ | +++ | ++ | +++ | ++ |
| Ceftazidime + Example 4 Compound | ++ | ++ | ++ | ++ | ++ |
| Ceftazidime + Example 5 Compound | ++ | ++ | + | + | - |
| Ceftazidime + Example 6 Compound | ++ | ++ | + | + | - |
| Ceftazidime + Example 7 Compound | ++ | ++ | + | + | - |
| Ceftazidime + Example 8 Compound | ++ | ++ | + | - | - |
| Ceftazidime + Example 9 Compound | ++ | ++ | + | - | - |
| Ceftazidime + Example 10 Compound | ++ | ++ | + | - | - |
| Ceftazidime + Example 11 Compound | ++ | ++ | + | - | - |
| Ceftazidime + Example 12 Compound | ++ | ++ | + | - | - |
| Ceftazidime + Example 13 Compound | ++ | ++ | + | - | - |
| Ceftazidime + Example 14 Compound | ++ | ++ | ++ | ++ | - |
| Ceftazidime + Example 15 Compound | ++ | ++ | ++ | ++ | - |
| Ceftazidime + Example 16 Compound | ++ | ++ | + | - | - |
| Ceftazidime + Example 17 Compound | ++ | ++ | + | - | - |
| Ceftazidime + Example 18 Compound | ++ | ++ | + | - | - |
| Ceftazidime + Example 19 Compound | ++ | ++ | + | - | - |
| Ceftazidime + Example 20 Compound | +++ | +++ | ++ | +++ | + |
| Ceftazidime + Example 21 Compound | +++ | +++ | ++ | +++ | + |
| Ceftazidime + Example 22 Compound | ++ | ++ | + | - | - |
| Ceftazidime + Example 23 Compound | ++ | ++ | + | - | - |
| Ceftazidime + Example 24 Compound | ++ | ++ | + | - | - |
| Ceftazidime + Example 25 Compound | ++ | ++ | + | - | - |
| Ceftazidime + Example 26 Compound | ++ | ++ | + | - | - |

| | | | | | |
|---|---|---|---|---|---|
| Note: "+++" indicates MIC₅₀ ≤ 0.1 mg/L; "++" indicates 0.1 mg/L < MIC₅₀ ≤ 1 mg/L; "+" indicates 1 mg/L < MIC₅₀ ≤ 10 mg/L; "-" indicates MIC₅₀ > 10 mg/L. | | | | | |

It can be seen from Table 1 above that when multiple Example compounds were used in combination with the β-lactam antibiotic ceftazidime, the MIC of ceftazidime against several β-lactamase-producing resistant bacteria was reduced several-fold, indicating that the Example compounds can effectively enhance the sensitivity of β-lactamase-producing resistant bacteria to β-lactam antibiotics. Among them, compared to avibactam, Example 2, Example 3, Example 20, and Example 21 were more effective in enhancing the antibacterial activity of ceftazidime against different Gram-negative bacteria, and particularly significantly enhanced the activity of ceftazidime against various Enterobacteriaceae bacteria expressing different β-lactamases.

**Table 2. Antibacterial activity (MIC₅₀, mg/L) of Example compounds in combination with different antibiotics**

| Strain Name | E. coli ATCC 25922 | E. coli ATCC 35218 | E. Coli NCTC 13353 | K.Pneumonia e BAA-1705 |
|---|---|---|---|---|
| Ceftazidime/Example 3 Compound | +++ | +++ | +++ | ++ |
| Aztreonam/Example 3 Compound | +++ | +++ | +++ | ++ |
| Meropenem/Example 3 Compound | +++ | +++ | +++ | ++ |
| Cefepime/Example 3 Compound | +++ | +++ | +++ | ++ |
| Ceftazidime/Example 20 Compound | +++ | +++ | +++ | + |
| Cefepime/Example 20 Compound | +++ | +++ | +++ | ++ |
| Imipenem/Relebactam | ++ | ++ | ++ | + |
| Ceftazidime/Avibactam | ++ | ++ | ++ | ++ |

| | | | | |
|---|---|---|---|---|
| Note: "+++" indicates MIC₅₀ ≤ 0.1 mg/L; "++" indicates 0.1 mg/L < MIC₅₀ ≤ 1 mg/L; "+" indicates 1 mg/L < MIC₅₀ ≤ 10 mg/L; "-" indicates MIC₅₀ > 10 mg/L. | | | | |

It can be seen from Table 2 above that when the Example compounds were used in combination with different β-lactam antibiotics (ceftazidime, aztreonam, meropenem, cefepime), the MIC of these antibiotics against several β-lactamase-producing resistant bacteria was reduced several-fold, indicating that the Example compounds can effectively enhance the sensitivity of β-lactamase-producing resistant bacteria to β-lactam antibiotics. Compared to the avibactam/ceftazidime combination or the relebactam/imipenem combination, Example 3 and Example 20, when combined with ceftazidime, aztreonam, meropenem, or cefepime, exhibited better antibacterial activity against various resistant Gram-negative bacteria. This suggests that the Examples of the present invention can be developed into drug combination regimens with a variety of β-lactam antibiotics for the treatment of infections caused by resistant Gram-negative bacteria.

**Table 3. Antibacterial activity (MIC₅₀, mg/L) of Example compounds in combination with different antibiotics against clinical resistant strains**

| Bacterial Species | Strain Name | β-Lactamase Expressed by Strain | Ceftazidime /Example 3 Compound | Cefepime/Example 3 Compound | Ceftazidime /Example 20 Compoun d | Ceftazidime /Avibactam |
|---|---|---|---|---|---|---|
| E. coli | 11119 | KPC-2 | +++ | +++ | ++ | + |
| | HX005215 | NDM-5 | +++ | +++ | +++ | - |
| | 14175 | KPC-2 | +++ | +++ | ++ | - |
| *K. pneumoniae* | HX085026 | KPC-2 | +++ | +++ | ++ | ++ |
| | HX085072 | KPC-2 | +++ | +++ | ++ | ++ |
| | HX085104 | KPC-2 | +++ | +++ | ++ | ++ |
| | HX090045 | KPC-2 | + | + | ++ | + |
| *E. cloacae* | 14653 | KPC-2/ NDM-1 | +++ | +++ | ++ | - |
| | CRE01 | NDM-1 | +++ | +++ | ++ | - |
| *P. aeruginosa* | C1475 | NDM-1 | + | - | + | + |

| | | | | | | |
|---|---|---|---|---|---|---|
| Note: "+++" indicates MIC₅₀ ≤ 0.1 mg/L; "++" indicates 0.1 mg/L < MIC₅₀ ≤ 1 mg/L; "+" indicates 1 mg/L < MIC₅₀ ≤ 10 mg/L; "-" indicates MIC₅₀ > 10 mg/L. | | | | | | |

It can be seen from Table 3 above that when the compound of Example 3 or the compound of Example 20 was used in combination with different β-lactam antibiotics (ceftazidime or cefepime), the MIC of these antibiotics against bacteria resistant due to the production of β-lactamases (including the serine hydrolase KPC-2 and the metallo-β-lactamases NDM-1, NDM-5) was reduced several-fold, which was significantly superior to the avibactam/ceftazidime combination. This indicates that the Example compounds can effectively enhance the sensitivity of β-lactamase-producing resistant bacteria to β-lactam antibiotics.

### Test Example 2: β-Lactamase Inhibitory Activity Test

The IC₅₀ of β-lactamase inhibitors against β-lactamases was tested using nitrocefin as the substrate. The enzyme and serially diluted β-lactamase inhibitor were incubated in 50 mM phosphate buffer (containing 0.1 mg/mL bovine serum albumin) at 37°C for 10 minutes. The reaction was initiated by adding 100 µM nitrocefin, and the reaction temperature was set to 37°C. The absorbance at 490 nm was recorded every minute for a total of 1 hour. The initial reaction rate was calculated, and the IC₅₀ was calculated using GraphPad Prism software.

**Table 4. Inhibitory activity (IC50, mg/L) of compounds against β-lactamases**

| β-Lactamase | **TEM-1** | **KPC-2** | **AmpC** | **OXA-10** | **OXA-23** |
|---|---|---|---|---|---|
| Enzyme Family | A | A | C | D | D |
| Example 1 Compound | +++ | +++ | +++ | +++ | +++ |
| Example 2 Compound | +++ | +++ | +++ | +++ | +++ |
| Example 3 Compound | +++ | +++ | +++ | +++ | +++ |
| Example 4 Compound | +++ | +++ | +++ | NT | ++ |
| Example 10 Compound | +++ | +++ | +++ | NT | +++ |
| Example 11 Compound | +++ | +++ | +++ | NT | ++ |
| Example 13 Compound | +++ | +++ | +++ | NT | + |
| Example 14 Compound | +++ | +++ | +++ | NT | + |
| Example 15 Compound | - | - | - | NT | - |

| | | | | | |
|---|---|---|---|---|---|
| Note: "+++" indicates IC₅₀ ≤ 1 µM; "++" indicates 1 µM < IC₅₀ ≤ 10 µM; "+" indicates 10 µM < IC₅₀ ≤ 100 µM; "-" indicates IC₅₀ > 100 µM; "NT" indicates not tested. | | | | | |

It can be seen from the table above that the Example compounds exhibited good inhibitory activity against all five selected β-lactamases from three classes (A, C, and D), suggesting that the compounds of the present invention can enhance bacterial sensitivity to existing β-lactam antibiotics by inhibiting β-lactamases.

From the above examples and experimental examples, it can be seen that the present invention provides a class of β-lactamase inhibitors. When used in combination with traditional β-lactam antibiotics, they can reverse the resistance of bacteria to β-lactam antibiotics caused by the expression of β-lactamases, showing promising application prospects in the clinical treatment of bacterial infections.

## Claims

1. A compound of Formula I, or a pharmaceutically acceptable salt, a stereoisomer, or a solvate thereof, 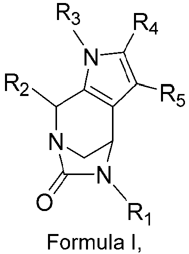 wherein:
R₁ is 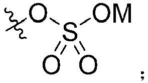
R₂ is selected from the group consisting of hydrogen, halogen, cyano, monofluoromethyl, difluoromethyl, trifluoromethyl, -SO₂C₁₋₆ alkyl, -SO₂C₃₋₆ cycloalkyl, -SO₂ aryl, -SO₂ heteroaryl, - C(O)NR₇R₈, -CH₂NR₇R₈, -C(NH)NR₇R₈, -C(O)OR₉, -C(NH)OR₉, -C(O)NR₁₀NR₇R₈, - C(O)NR₁₀NR₁₁C(O)R₁₂, -C(O)NR₁₀OR₉, -C(NH)NR₁₀OR₉, -C(O)NR₁₀(CH₂)mNR₇R₈, - C(O)NR₁₀(CH₂)_{M}NR₁₁C(O)R₁₂, and -CH₂NR₇C(O)R₉;
R₃ is selected from the group consisting of hydrogen, C₁₋₆ alkyl, C₃₋₆ cycloalkyl, C₄₋₇ heterocycloalkyl, C₃₋₆ cycloketone, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and - C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl, heterocycloalkyl, and cycloketone are optionally substituted with zero, one, or more substituents each independently selected from the group consisting of C₁₋₆ alkyl, hydroxyl, halogen, and -NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and -C(O)NR₁₅R₁₆;
wherein the aryl and heteroaryl of R₄ and R₅ are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₃ and R₁₄ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₅ and R₁₆ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
m is 1, 2, 3 or 4; and
M is selected from the group consisting of hydrogen, a metal ion, and an organic cation.

2. The compound according to claim 1, or a pharmaceutically acceptable salt, a stereoisomer, or a solvate thereof, wherein:
R₂ is selected from the group consisting of hydrogen, cyano, -C(O)NR₇R₈, -CH₂NR₇R₈, - C(NH)NR₇R₈, -C(O)OR₉, -C(NH)OR₉, -C(O)NR₁₀NR₇R₈, -C(O)NR₁₀NR₁₁C(O)R₁₂, - C(O)NR₁₀OR₉, -C(NH)NR₁₀OR₉, -C(O)NR₁₀(CH₂)mNR₇R₈, -C(O)NR₁₀(CH₂)mNR₁₁C(O)R₁₂, and -CH₂NR₇C(O)R₉;
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring containing one or two heteroatoms optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl; and m is 1, 2, 3, or 4.

3. The compound according to claim 1, or a pharmaceutically acceptable salt, a stereoisomer, or a solvate thereof, wherein the compound is of Formula I-A: 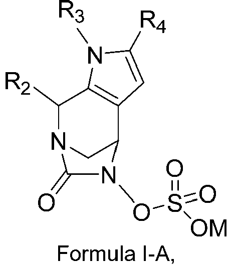 wherein,
R₂ is selected from the group consisting of hydrogen, halogen, cyano, monofluoromethyl, difluoromethyl, trifluoromethyl, -C(O)NR₇R₈, -CH₂NR₇R₈, -C(O)OR₉, and -CH₂NR₇C(O)R₉;
R₃ is selected from the group consisting of hydrogen, C₁₋₆ alkyl, C₃₋₆ cycloalkyl, -C(O)C₁₋₆ alkyl, -C(O)C₃₋₆ cycloalkyl, -C(O)C₂₋₆ alkenyl, -C(O) phenyl, -C(O) pyrazolyl, -C(O)NR₁₃R₁₄, - SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and -NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆; wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, and piperidinyl;
R₉ is selected from the group consisting of hydrogen and C₁₋₆ alkyl;
R₁₃, R₁₄, R₁₅, and R₁₆ are each independently selected from the group consisting of hydrogen and C₁₋₆ alkyl; and M is selected from the group consisting of hydrogen and a sodium ion.

4. The compound according to claim 3, or a pharmaceutically acceptable salt, a stereoisomer, or a solvate thereof, wherein the compound is of Formula I-B: 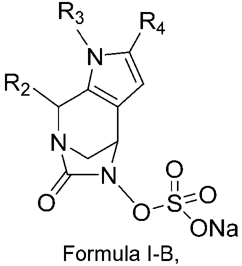 wherein,
R₂ is selected from the group consisting of hydrogen, halogen, cyano, monofluoromethyl, difluoromethyl, trifluoromethyl, -C(O)NR₇R₈, -CH₂NR₇R₈, -C(O)OR₉, and -CH₂NR₇C(O)R₉;
R₃ is selected from the group consisting of hydrogen, C₁₋₆ alkyl, C₃₋₆ cycloalkyl, -C(O)C₁₋₆ alkyl, -C(O)C₃₋₆ cycloalkyl, -C(O)C₂₋₆ alkenyl, -C(O) phenyl, -C(O) pyrazolyl, -C(O)NR₁₃R₁₄, - SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and -NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆;
wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, and piperidinyl;
R₉ is selected from the group consisting of hydrogen and C₁₋₆ alkyl; and R₁₃, R₁₄, R₁₅, and R₁₆ are each independently selected from the group consisting of hydrogen and C₁₋₆ alkyl.

5. The compound according to claim 4, or a pharmaceutically acceptable salt, a stereoisomer, or a solvate thereof, wherein the compound is of Formula I-C: 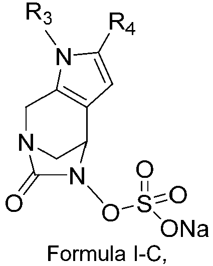 wherein,
R₃ is selected from the group consisting of hydrogen, C₁₋₆ alkyl, C₃₋₆ cycloalkyl, -C(O)C₁₋₆ alkyl, -C(O)C₃₋₆ cycloalkyl, -C(O)C₂₋₆ alkenyl, -C(O) phenyl, -C(O) pyrazolyl, -C(O)NR₁₃R₁₄, - SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄;
wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄;
wherein the cycloalkyl is optionally substituted with zero, one, or more substituents each independently selected from the group consisting of C₁₋₆ alkyl, hydroxyl, halogen, and -NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆;
wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆; and R₁₃, R₁₄, R₁₅, and R₁₆ are each independently selected from the group consisting of hydrogen and C₁₋₆ alkyl.

6. The compound according to any one of claims 1-5, or a pharmaceutically acceptable salt, a stereoisomer, or a solvate thereof, wherein the compound is one of the following compounds:

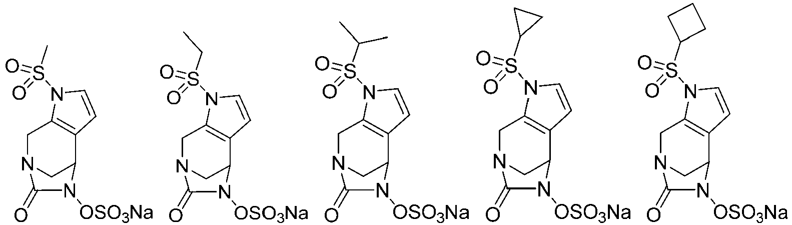

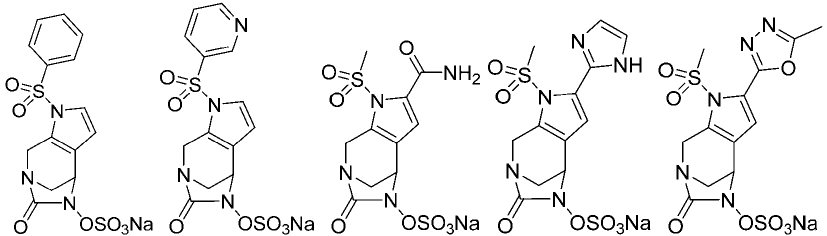

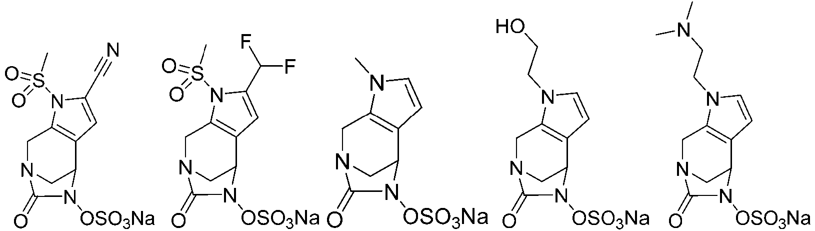

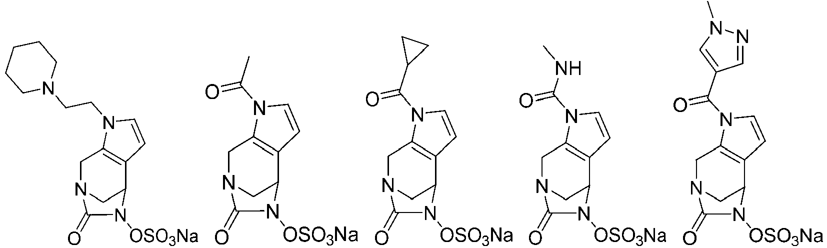

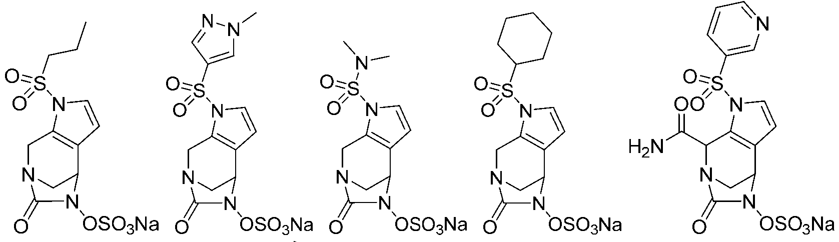

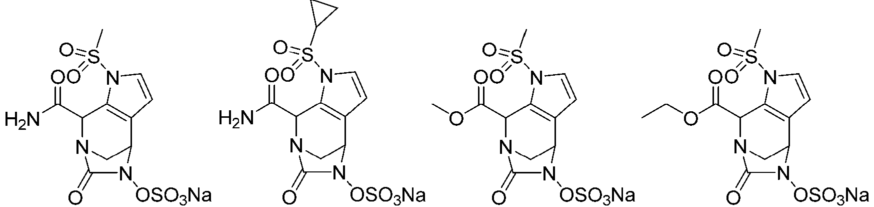

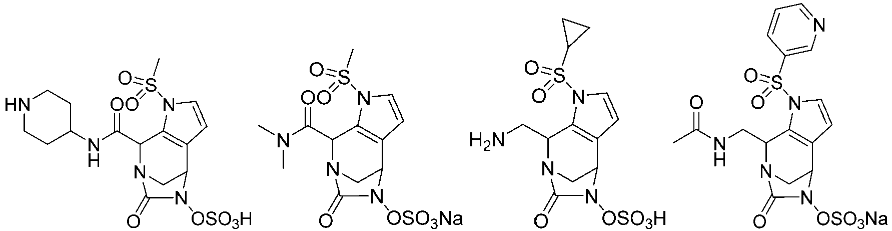

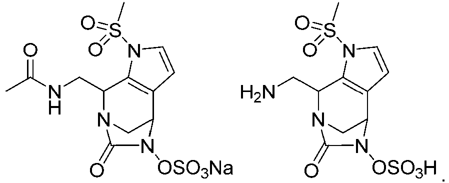

7. The compound according to claim 1, or a pharmaceutically acceptable salt, a stereoisomer, or a solvate thereof, wherein the compound is represented by Formula II, 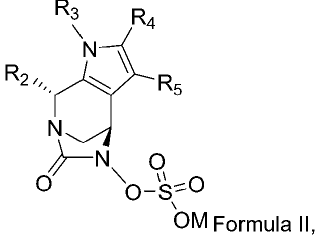 wherein:
R₂ is selected from the group consisting of hydrogen, halogen, cyano, monofluoromethyl, difluoromethyl, trifluoromethyl, -SO₂C₁₋₆ alkyl, -SO₂C₃₋₆ cycloalkyl, -SO₂ aryl, -SO₂ heteroaryl, - C(O)NR₇R₈, -CH₂NR₇R₈, -C(NH)NR₇R₈, -C(O)OR₉, -C(NH)OR₉, -C(O)NR₁₀NR₇R₈, - C(O)NR₁₀NR₁₁C(O)R₁₂, -C(O)NR₁₀OR₉, -C(NH)NR₁₀OR₉, -C(O)NR₁₀(CH₂)mNR₇R₈, - C(O)NR₁₀(CH₂)mNR₁₁C(O)R₁₂, and -CH₂NR₇C(O)R₉;
R₃ is selected from the group consisting of hydrogen, C₁₋₆ alkyl, C₃₋₆ cycloalkyl, C₄₋₇ heterocycloalkyl, C₃₋₆ cycloketonyl, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and - C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and
wherein the cycloalkyl, heterocycloalkyl, and cycloketonyl are optionally substituted with zero, one, or more substituents each independently selected from the group consisting of C₁₋₆ alkyl, hydroxyl, halogen, and -NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and -C(O)NR₁₅R₁₆; wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₇ and R₈ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₇ and R₈ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
R₉ is selected from the group consisting of hydrogen, C₁₋₆ alkyl, C₃₋₆ cycloalkyl, C₄₋₇ heterocycloalkyl, aryl, and heteroaryl;
R₁₀ and R₁₁ are each independently selected from the group consisting of hydrogen, C₁₋₆ alkyl, C₃₋₆ cycloalkyl, C₄₋₇ heterocycloalkyl, aryl, and heteroaryl;
R₁₂ is selected from the group consisting of hydrogen, C₁₋₆ alkyl, C₃₋₆ cycloalkyl, C₄₋₇ heterocycloalkyl, aryl, and heteroaryl;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₃ and R₁₄ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₅ and R₁₆ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen;
m is 1, 2, 3, or 4; and
M is selected from the group consisting of hydrogen, a metal ion, and an organic cation.

8. The compound according to claim 7, or a pharmaceutically acceptable salt, a stereoisomer, or a solvate thereof, wherein the compound is of Formula III, 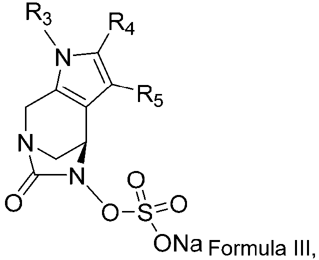 wherein:
R₃ is selected from the group consisting of hydrogen, C₁₋₆ alkyl, C₃₋₆ cycloalkyl, C₄₋₇ heterocycloalkyl, C₃₋₆ cycloketonyl, aryl, heteroaryl, -C(O)C₁₋₆ alkyl, -C(O)C₃₋₆ cycloalkyl, - C(O)C₂₋₆ alkenyl, -C(O) aryl, -C(O) heteroaryl, -C(O)NR₁₃R₁₄, -SO₂C₁₋₆ alkyl, -SO₂C₃₋₆ cycloalkyl, -SO₂ aryl, -SO₂ heteroaryl, and -SO₂NR₁₃R₁₄;
wherein the alkyl and alkenyl are optionally substituted with zero, one, or more substituents each independently selected from the group consisting of halogen, hydroxyl, C₃₋₆ cycloalkyl, C₄₋₇ heterocycloalkyl, C₂₋₆ alkenyl, C₂₋₆ alkynyl, aryl, heteroaryl, -NR₁₃R₁₄, -C(O)OC₁₋₆ alkyl, and - C(O)NR₁₃R₁₄;
wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and
wherein the cycloalkyl, heterocycloalkyl, and cycloketonyl are optionally substituted with zero, one, or more substituents each independently selected from the group consisting of C₁₋₆ alkyl, hydroxyl, halogen, and -NR₁₃R₁₄;
R₄ and R₅ are each independently selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, aryl, heteroaryl, -C(O)OC₁₋₆ alkyl, and -C(O)NR₁₅R₁₆; wherein the aryl and heteroaryl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆;
R₁₃ and R₁₄ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₃ and R₁₄ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen; and
R₁₅ and R₁₆ are each independently selected from the group consisting of hydrogen, C₁₋₆ alkyl, C₃₋₆ cycloalkyl, C₄₋₇ heterocycloalkyl, aryl, heteroaryl, -SO₂C₁₋₆ alkyl, -SO₂C₃₋₆ cycloalkyl, -SO₂ aryl, -SO₂ heteroaryl, -C(O)C₁₋₆ alkyl, -C(O)C₃₋₆ cycloalkyl, -C(O) aryl, and -C(O) heteroaryl; or R₁₅ and R₁₆ together with the nitrogen atom to which they are attached form a saturated ring comprising one or two heteroatoms, wherein the saturated ring is optionally substituted with zero, one, or more substituents each independently selected from the group consisting of hydrogen, C₁₋₆ alkyl, hydroxyl, and halogen.

9. The compound according to claim 8, or a pharmaceutically acceptable salt, a stereoisomer, or a solvate thereof, wherein the compound is of Formula IV, 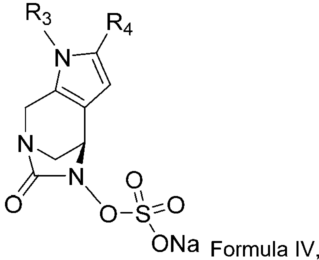 wherein:
R₃ is selected from the group consisting of hydrogen, C₁₋₆ alkyl, C₃₋₆ cycloalkyl, -C(O)C₁₋₆ alkyl, -C(O)C₃₋₆ cycloalkyl, -C(O)C₂₋₆ alkenyl, -C(O) phenyl, -C(O) pyrazolyl, -C(O)NR₁₃R₁₄, - SO₂C₁₋₆ alkyl, -SO₂C₃₋₆ cycloalkyl, -SO₂ phenyl, -SO₂ pyridyl, -SO₂ pyrazolyl, and -SO₂NR₁₃R₁₄; wherein the alkyl and alkenyl are optionally substituted with zero, one, or more substituents each independently selected from the group consisting of halogen, hydroxyl, C₃₋₆ cycloalkyl, piperidinyl, C₂₋₆ alkenyl, C₂₋₆ alkynyl, -NR₁₃R₁₄, -C(O)OC₁₋₆ alkyl, and -C(O)NR₁₃R₁₄; wherein the pyrazolyl, phenyl, and pyridyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₃R₁₄; and wherein the cycloalkyl is optionally substituted with zero, one, or more substituents each independently selected from the group consisting of C₁₋₆ alkyl, hydroxyl, halogen, and -NR₁₃R₁₄;
R₄ is selected from the group consisting of hydrogen, halogen, monofluoromethyl, difluoromethyl, trifluoromethyl, C₃₋₆ cycloalkyl, C₂₋₆ alkenyl, C₂₋₆ alkynyl, carboxyl, acetyl, cyano, cyanoethyl, phenyl, imidazolyl, oxadiazolyl, -C(O)OC₁₋₆ alkyl, and -C(O)NR₁₅R₁₆; wherein the phenyl, imidazolyl, and oxadiazolyl are optionally substituted with zero, one, or more substituents each independently selected from the group consisting of halogen, C₁₋₆ alkyl, C₃₋₆ cycloalkyl, C₂₋₆ alkenyl, C₂₋₆ alkynyl, -OC₁₋₆ alkyl, and -NR₁₅R₁₆; and
R₁₃, R₁₄, R₁₅, and R₁₆ are each independently selected from the group consisting of hydrogen and C₁₋₆ alkyl.

10. The compound according to any one of claims 7-9, or a pharmaceutically acceptable salt, a stereoisomer, or a solvate thereof, wherein the compound is one of the following compounds:

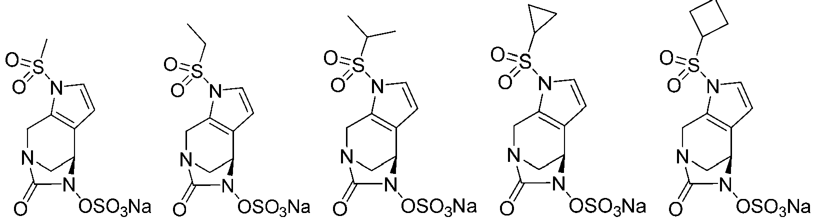

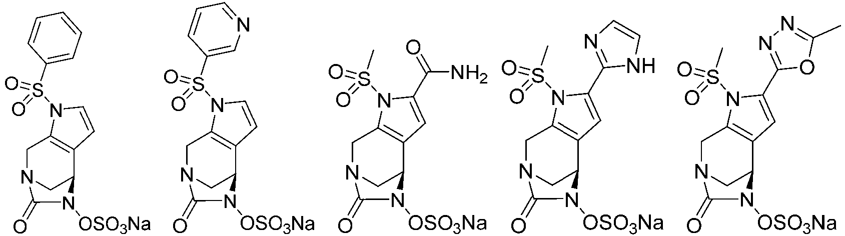

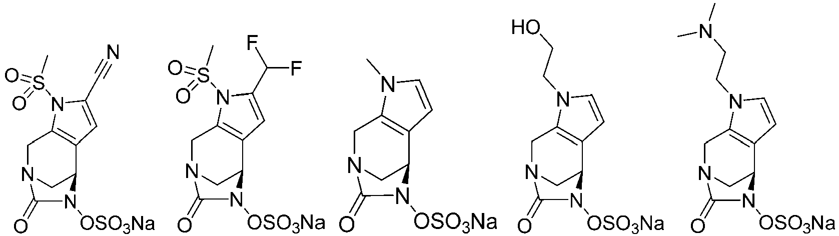

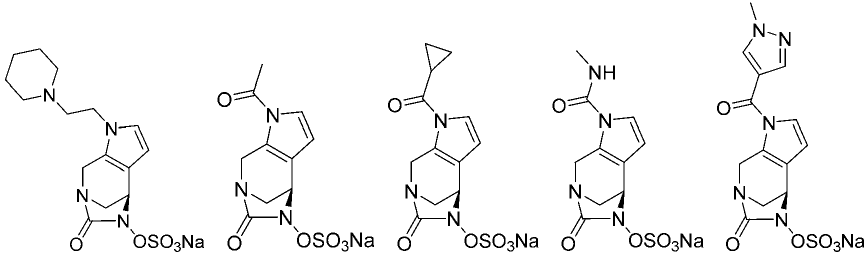

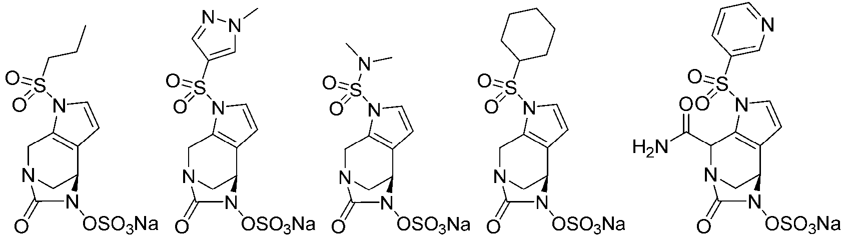

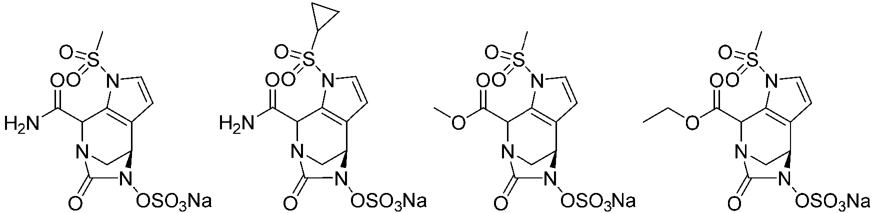

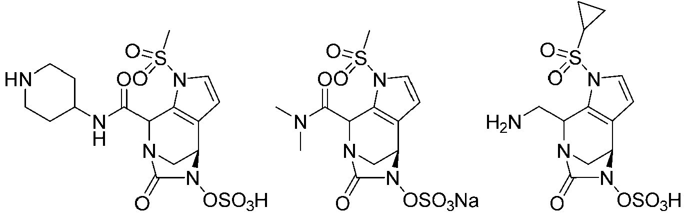

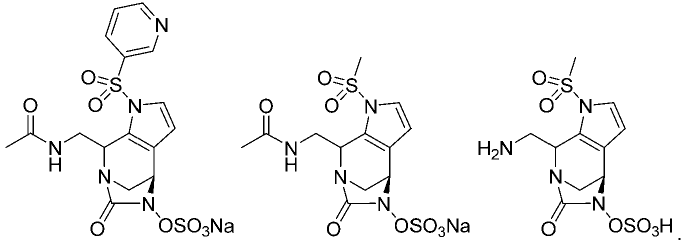

11. A method for preparing the compound according to any one of claims 1-10, comprising: 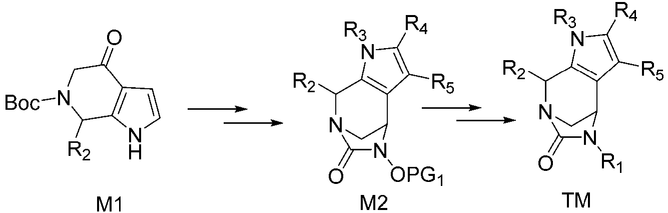
Step 1: subjecting a bicyclic compound M1 to a reduction reaction, a Mitsunobu reaction, a substitution reaction, a deprotection reaction, and a cyclization reaction to obtain a tricyclic intermediate M2;
Step 2: subjecting the tricyclic intermediate M2 to a deprotection reaction, a substitution reaction, and an ion-exchange reaction to obtain a target compound TM;
wherein R₁, R₂, R₃, R₄, and R₅ are as defined in any one of claims 1-10; and PG₁ is selected from hydroxyl protecting groups.

12. The method according to claim 11, wherein the preparation method of compound M1 comprises: 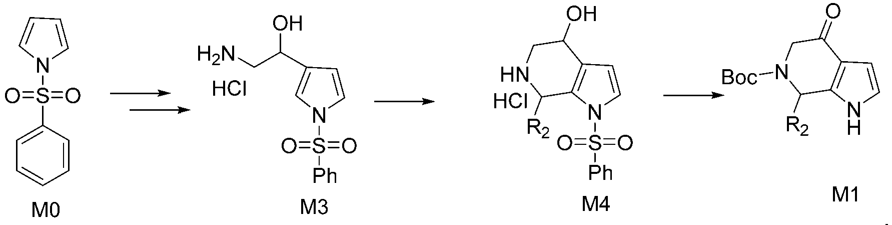
subjecting a compound M0 to a Friedel-Crafts reaction, a substitution reaction, and a reduction reaction to obtain an intermediate M3;
cyclizing the intermediate M3 to obtain a bicyclic intermediate M4; and
subjecting the bicyclic intermediate M4 to a protection reaction, an oxidation reaction, and a deprotection reaction to obtain the intermediate M1;
wherein R₂ is as defined in any one of claims 1-10.

13. Use of the compound according to any one of claims 1-10, or a pharmaceutically acceptable salt, a stereoisomer, or a solvate thereof, in the preparation of a β-lactamase inhibitor.

14. Use of the compound according to any one of claims 1-10, or a pharmaceutically acceptable salt, a stereoisomer, or a solvate thereof, in the preparation of a medicament for treating bacterial infection.

15. The use according to claim 14, wherein the medicament is for treating a drug-resistant bacterial infection expressing β-lactamase.

16. Use of the compound according to any one of claims 1-10, or a pharmaceutically acceptable salt, a stereoisomer, or a solvate thereof, in the preparation of a medicament for enhancing bacterial sensitivity to antibiotics.

17. The use according to claim 16, wherein the medicament enhances the sensitivity of β-lactamase-producing drug-resistant bacteria to β-lactam antibiotics.

18. A medicament for treating bacterial infection and/or enhancing bacterial sensitivity to antibiotics, wherein the medicament comprises, as an active ingredient, the compound according to any one of claims 1-10, or a pharmaceutically acceptable salt, a stereoisomer, or a solvate thereof, together with one or more pharmaceutically acceptable excipients or auxiliary ingredients.

19. The medicament according to claim 18, wherein the medicament is for treating a drug-resistant bacterial infection expressing β-lactamase and/or enhances the sensitivity of β-lactamase-producing drug-resistant bacteria to β-lactam antibiotics.

20. A combination medicament for treating bacterial infection, wherein the combination medicament comprises the medicament according to claim 18 or 19 and at least one β-lactam antibiotic.

21. A pharmaceutical composition for treating bacterial infection, wherein the pharmaceutical composition comprises, as an active ingredient, the combination medicament according to claim 20 together with one or more pharmaceutically acceptable excipients or auxiliary ingredients.
